# FASCICULE DE BREVET EUROPEEN

(11) **EP 1 077 928 B1**
(45) Date de publication et mention de la délivrance du brevet: **07.12.2005**
(21) Numéro de dépôt: 99918037.5
(22) Date de dépôt: 10.05.1999
(51) Int. Cl.: C07C 233/43, C07C 233/62, C07C 237/48, C07C 271/30, C07C 275/24, C07C 311/37, C07D 307/81, C07D 307/85, C07D 311/58, C07D 319/18, C07D 333/58, C07D 471/04, A61K 31/165

(54) **NOUVEAUX COMPOSES CYCLIQUES SUBSTITUES, LEUR PROCEDE DE PREPARATION ET LES COMPOSITIONS PHARMACEUTIQUES QUI LES CONTIENNENT**
SUSTIUIERTE CYCLISCHE VERBINDUNGEN, VERFAHREN ZU IHRER HERSTELLUNG SOWIE SIE ENTHALTENDE PHARMAZEUTISCHE ZUSAMMESETZUNGEN
NOVEL SUBSTITUTED CYCLIC COMPOUNDS, PREPARATION METHOD AND PHARMACEUTICAL COMPOSITIONS CONTAINING THEM

(30) Priorité: 12.05.1998 FR 9805957
(43) Date de publication de la demande: 28.02.2001
(73) Titulaire: Les Laboratoires Servier, 92200 Neuilly sur Seine (FR)
(72) Inventeur: LESIEUR, Daniel, F-59147 Gondecourt (FR); KLUPSCH, Frédérique, F-62640 Montigny en Gohelle (FR); GUILLAUMET, Gérald, F-45650 Saint Jean le Blanc (FR); VIAUD, Marie-Claude, F-45000 Orléans (FR); LANGLOIS, Michel, F-92330 Sceaux (FR); BENNEJEAN, Caroline, F-94220 Charenton Le Pont (FR); RENARD, Pierre, F-78150 Le Chesnay (FR); DELAGRANGE, Philippe, F-92130 Issy les Moulineaux (FR)
(86) Numéro de dépôt international: PCT/FR1999/001101
(87) Numéro de publication internationale: WO 1999/058496

(56) Documents cités:
- EP-A- 0 530 087
- EP-A- 0 562 956
- EP-A- 0 662 471
- EP-A- 0 728 738
- EP-A- 0 745 583
- EP-A- 0 745 584

## Description

La présente invention concerne de nouveaux dérivés cycliques substitués, leur procédé de préparation et les compositions pharmaceutiques qui les contiennent.

On connaît, dans l'art antérieur, des indoles à chaîne rétroamide substitués par des amides ou des carbamates utiles en tant qu'antagonistes de la GnRH (WO 9721707) et des indoles à chaîne amide substitués par des amides, carbamates ou urées utiles en tant qu'agents antihypertenseurs (US 4803218).

Des composés benzofuraniques ou benzothiophéniques à chaîne rétroamide substitués par des amides ou carbamates sont également décrits en tant qu'agents anti-inflammatoires (EP 685475) ou inhibiteurs de la résorption osseuse.

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.
Outre leur action bénéfique sur les troubles du rythme circadien (J. Neurosurg. 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Sécrétions, 1990, 8 (3-4), pp 264-272) et analgésiques (Pharmacopsychiat., 1987, 20, pp 222-223) ainsi que pour le traitement des maladies de Parkinson (J. Neurosurg. 1985, 63, pp 321-341) et d'Alzheimer (Brain Research, 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720), sur le diabète (Clinical Endocrinology, 1986, 24, pp 359-364), et dans le traitement de l'obésité (International Journal of Eating Disorders, 1996, 20 (4), pp 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p 50 ; WO 97.04094). Certains de ces récepteurs ont pu être localisés et caractérisés pour différentes espèces, dont les mammifères.
Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sous-types réceptoriels mélatoninergiques.

La présente invention concerne plus particulièrement les composés de formule (I) :

R-A-R' (I)

dans laquelle :
◆ A représente :
   - un système cyclique de formule (II) : 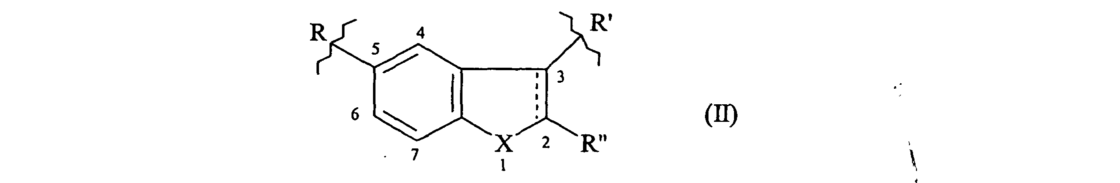 où
      - X représente un atome d'oxygène ou de soufre, ou un groupement NR₀ (dans lequel R₀ représente un atome d'hydrogène, un groupement alkyle (C₁-C₆) linéaire ou ramifié, aryle, arylalkyle (C₁-C₆) linéaire ouramifié ou SO₂Ph),
      - la représentation ... signifie que la liaison peut être simple ou double étant entendu que la valence des atomes est respectée,
      - R" représente un groupement Rₐ, ORₐ, CORₐ, COORₐ, OCORₐ, OSO₂CF₃ ou atomes d'halogène,
         où Rₐ représente un atome d'hydrogène, un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, polyhalogénoalkyle (C₁-C₆) linéaire ou ramifié, cycloalkyle (C₃-C₈) substitué ou non, cycloalkyle (C₃-C₈) alkyle (C₁-C₆) linéaire ou ramifié substitué ou non, cycloalkényle (C₃-C₈) substitué ou non, cycloalkényle (C₃-C₈) alkyle (C₁-C₆) linéaire ou ramifié substitué ou non, aryle, arylalkyle (C₁-C₆) linéaire ou ramifié, arylalkényle (C₁-C₆) linéaire ou ramifié, hétéroaryle, hétéroarylalkyle (C₁-C₆) linéaire ou ramifié, hétéroarylalkényle (C₁-C₆) linéaire ou ramifié, hétérocycloalkyle substitué ou non, hétérocycloalkényle substitué ou non, hétérocycloalkylalkyle (C₁-C₆) linéaire ou ramifié substitué ou non, ou hétérocycloalkényl alkyle (C₁-C₆) linéaire ou ramifié substitué ou non,
   - ou un système cyclique de formule (III) : 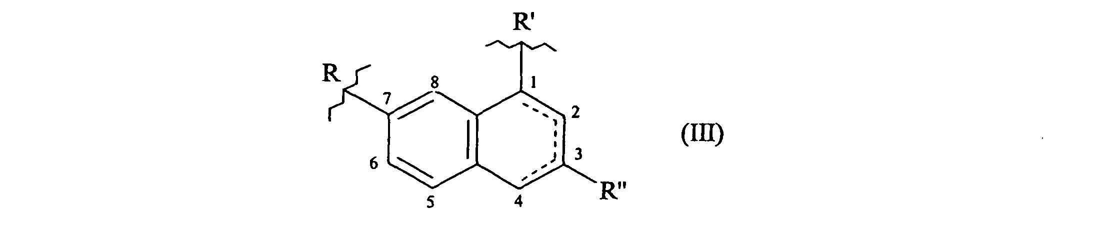 où R" et la représentation .... ont la même signification que précédemment,
◆ R représente :
   un groupement de formule (VI) : 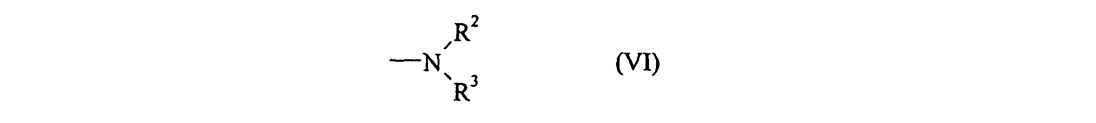 où
   - R² représente un groupement Rₐ tel que défini précédemment,
   - R³ représente un groupement COR'ₐ, CSR'ₐ, CONR'ₐR"ₐ, CSNR'ₐR"ₐ, COOR'ₐ, CSOR'ₐ ou S(O)ᵥR'ₐ (dans lesquels R'ₐ et R"ₐ, identiques ou différents, peuvent prendre toutes les valeurs de Rₐ défini précédemment et peuvent également former, avec l'atome d'azote qui les porte un groupement cyclique contenant 5 à 10 chaînons et pouvant comporter, en plus de l'atome d'azote par lequel il est relié, un à trois hétéroatomes choisis parmi oxygène, soufre et azote, et v vaut 1 ou 2),
◆ et R' représente un groupement de formule (IX) :

   ― G ― R⁵ (IX)

   où
   - G représente une chaîne alkylène -(CH₂)ₜ- (dans laquelle t vaut 2 ou 3)
   - et R⁵ représente un groupement 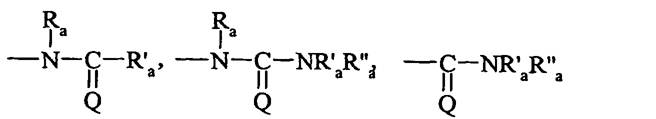 ou 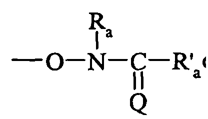 dans lesquels Q représente un atome d'oxygène ou de soufre, et Rₐ, R'ₐ et R"ₐ (identiques ou différents) sont définis de la même façon que précédemment, R'ₐ et R"ₐ pouvant former avec l'atome d'azote qui les porte un groupement cyclique tel que défini précédemment,
   étant entendu que :
   ― par "hétérocycloalkyle" on entend tout groupement saturé mono ou polycyclique contenant de 5 à 10 atomes contenant 1 à 3 hétéroatomes choisis parmi azote, oxygène ou soufre,
   ― par "hétérocycloalkényle" on entend tout groupement mono ou polycyclique non aromatique contenant une ou plusieurs insaturations, contenant de 5 à 10 atomes et pouvant contenir 1 à 3 hétéroatomes choisis parmi azote, oxygène ou soufre,
   ― le terme "substitué" affecté aux expressions "alkyle", "alkényle", "alkynyle", signifie que ces groupements sont substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
   ― le terme "substitué" affecté aux expressions "cycloalkyle", "cycloalkylalkyle", "cycloalkényle", "cycloalkénylalkyle", "hétérocycloalkyle", "hétérocycloalkényle", "hétérocycloalkylalkyle", "hétérocycloalkénylalkyle", signifie que la partie cyclique de ces groupements est substituée par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
   ― par "aryle" on entend tout groupement aromatique mono ou polycyclique contenant 6 à 22 atomes de carbone, ainsi que le groupement biphényle,
   ― par "hétéroaryle" on entend tout groupement aromatique mono ou polycyclique contenant de 5 à 10 atomes contenant 1 à 3 hétéroatomes choisis parmi azote, oxygène ou soufre,
      les groupements "aryle" et "hétéroaryle" pouvant être substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, cyano, carboxy, nitro, amino ou atomes d'halogène,
      étant entendu que :
   ― lorsque A représente un noyau indolique, R" représente un atome d'hydrogène,
   ― lorsque A représente un noyau indolique et R représente un groupement -NHCOR'ₐ, -NHCOOR'ₐ ou NHCONR'ₐR"ₐ, alors G-R⁵ ne peut représenter un groupement -(CH₂)₂-NHCOR_{b} dans lequel R_{b} représente un groupement alkyl (C₁-C₄) ou CF₃,
   ― lorsque A représente un noyau benzofurane ou benzothiophène, R" est différent d'un groupement COPh (où Ph est substitué ou non),
   ― lorsque A représente un noyau benzofurane ou benzothiophène, R ne peut représenter un groupement -NRₐCOR_{c}, -NHSO₂R_{c}, -NHCOCH₂R_{c}, ou NHCONHR_{c} où R_{c} représente un groupement hétérocyclique ou aryle,
   ― lorsque A représente un tétrahydronaphtalène, R⁵ ne peut représenter un groupement CONR'ₐR"ₐ tel que défini précédemment,
leurs énantiomères et diastéréoisomères, ainsi que leurs sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

De façon avantageuse, l'invention concerne les composés pour lesquels A représente un système cyclique de formule (II) comme par exemple (dihydro)benzothiophène, (dihydro)benzofurane, indole ou indoline,
ou de formule (III) comme par exemple naphtalène ou tétrahydronaphtalène.

De façon plus avantageuse, les substituants R préférés de l'invention sont ceux représentés par un groupement de formule (VI) dans lequel R³ représente un groupement COR'ₐ ou COOR'ₐ (où R'ₐ est tel que défini précédemment)

De façon encore plus avantageuse, les substituants R préférés de l'invention sont ceux représentés par un groupement NCOR'ₐ ou NCOOR'ₐ où R'ₐ représente un atome d'hydrogène, un groupement alkyle, polyhalogénoalkyle, alkényle, alkynyle, cycloalkyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthylcyclopropyle, furyle, thiényle, pyridyle, furylméthyle, pyridylméthyle.

Les substituants R' préférés de l'invention sont ceux pour lesquels G représente une chaîne alkylène -(CH₂)ₜ- où t vaut 2 ou 3, et R⁵ représente un groupement 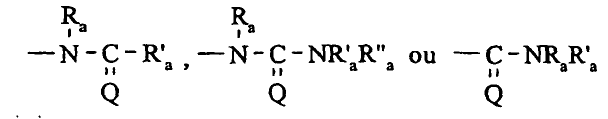 dans lesquels Rₐ, R'ₐ, R"ₐ et Q sont tels que définis précédemment.

De façon encore plus avantageuse, les substituants R' préférés de l'invention sont ceux pour lesquels G représente un groupement -(CH₂)ₜ- où t vaut 2 ou 3, et R⁵ représente un groupement 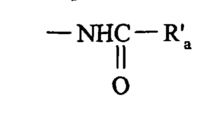 où R'ₐ représente un groupement alkyle, polyhalogénoalkyle, alkényle, alkynyle, cycloakyle, cycloalkényle, cycloakylalkyle, cycloalkénylalkyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthylcyclopropyle, furyle, thiényle, pyridyle, furylméthyle, pyridylméthyle,
ou G représente un groupement -(CH₂)₃- et R⁵ représente un groupement 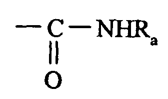 où Rₐ représente un groupement alkyle, polyhalogénoalkyle, alkényle, alkynyle, cycloakyle, cycloalkényle, cycloakylalkyle, cycloalkénylalkyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthylcyclopropyle, furyle, thiényle, pyridyle, furylméthyle, pyridylméthyle.

De façon encore plus avantageuse, les composés préférés de l'invention sont ceux pour lesquels
A représente un système cyclique de formule (II) ou (III),
R représente un groupement NHCOR'ₐ ou NHCOOR'ₐ (où R'ₐ est défini comme précédemment),
et R' est tel que G représente une chaîne alkylène -(CH₂)ₜ- non substituée ou substituée où t vaut 2 ou 3, et R⁵ représente un groupement 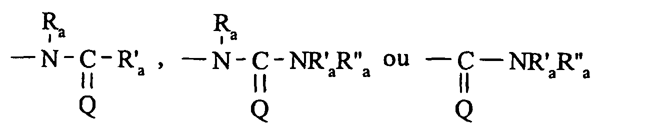 où Rₐ, R'ₐ, R"ₐ et Q sont tels que définis précédemment.

Encore plus particulièrement, l'invention concerne les composés de formule (I) pour lesquels A représente un (dihydro)benzothiophène, (dihydro)benzofurane, indole, indoline, dihydronaphtalène, tétrahydronaphtalène ou naphtalène, R représente un groupement NHCOR'ₐ ou NHCOOR'ₐ où R'ₐ représente un atome d'hydrogène, un groupement alkyle, polyhalogénoalkyle, alkényle, alkynyle, cycloalkyle, cycloalkényle, cycloalkylalkyle, cycloalkénylakyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthylcyclopropyle, furyle, thiényle, pyridyle, furylméthyle ou pyridylméthyle,
et R' représente un groupement -(CH₂)ₜ-NHCOR'ₐ où t vaut 2 ou 3 et R'ₐ représente un groupement alkyle, polyhalogénoalkyle, alkényle, alkynyle, cycloalkyle, cycloalkényle, cycloalkylalkyle, cycloalkénylalkyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthyle cyclopropyle, furyle, thiényle, pyridyle, furylméthyle, pyridylméthyle.

Encore plus avantageusement, les composés préférés de l'invention sont :
des naphtalènes, dihydronaphtalènes ou tétrahydronaphtalènes éventuellement substitués en position 3, substitués en 7 par un groupement NHCORₐ ou NHCOORₐ, (où Rₐ est défini comme précédemment), et substitués en 1 par un groupement -(CH₂)ₜ-NHCOR'ₐ où t vaut 2 ou 3 et R'ₐ est tel que défini précédemment,
ou des benzofurane ou benzothiophène éventuellement substitués en position 2, substitués en 5 par un groupement NHCORₐ ou NHCOORₐ, (où Rₐ est défini comme précédemment), et substitués en 3 par un groupement -(CH₂)ₜ-NHCOR'ₐ où t vaut 2 ou 3 et R'ₐ est défini comme précédemment.

L'invention concerne tout particulièrement les composés de formule (I) qui sont :
- le N-{3-[2-(Acétylamino)éthyl]-1-benzofuran-5-yl}-2,2,2-trifluoroacétamide,
- le N- {2-[5-(Acétylamino)-1-benzofuran-3-yl]éthyl}cyclopropane carboxamide,
- le N-{2-[5-(Acétylamino)-1-benzothiophèn-3-yl]éthyl}benzamide,
- le N-{8-[2-([2-Phénylacétyl]amino)éthyl]-2-naphtyl}butanamide,
- le N-(8-{2-[(2-Bromoacétyl)amino]éthyl}-2-naphtyl)-1-cyclohexanecarboxamide,
- le N-{8-[2-(Heptanoylamino)éthyl]-2,6-dinaphtyl}-2-butènamide,
- le N-{8-[2-(Acétylamino)éthyl]-2-naphtyl}acétamide,
- le N-{3-[2-(Acétylamino)éthyl]benzo[*b*]furan-5-yl}carbamate de méthyle,
- le 3-{2-[(Cyclopropylcarbonyl)amino]éthyl}-1-benzofuran-5-yl-carbamate de méthyle,
- le 3-[2-(Acétylamino)éthyl]-1-benzofuran-5-yl-carbamate de tert-butyle,
- le 3-[2-(Acétylamino)éthyl]-1-benzofuran-5-yl-(méthyl)carbamate de tert-butyle,
- le 3-[2-(Benzoylamino)éthyl)-1-benzofuran-5-yl carbamate de méthyle,
- le 3-[2-(Isobutyrylamino)éthyl]-1-benzofuran-5-yl carbamate de méthyle,
- le N-(8-{2-[(2-Bromoacétyl)amino]éthyl}-2-naphtyl)carbamate d'éthyle,
- le N-{8-[2-(Acétylamino)éthyl]-6-phényl-2-naphtyl}carbamate de méthyle,
- le N-{8-[2-(Acétylamino)éthyl]-5,6,7,8-tétrahydro-2-naphtalényl}carbamate d'hexyle,
- le 8-[2-(Acétylamino)éthyl]-2-naphtyl carbamate de méthyle
- le 3-[2-(2-Furoylamino)éthyl]-1-benzofuran-5-yl carbamate de méthyle,
- le 3-{2-[(Cyclopentylcarbonyl)amino]éthyl}-1-benzofuran-5-yl carbamate de méthyle.
   Les énantiomères, diastéréoisomères ainsi que les sels d'addition à un acide ou une base pharmaceutiquement acceptable des composés préférés de l'invention font partie intégrante de l'invention.
   L'invention s'étend également au procédé de préparation des composés de formule (I) caractérisé en ce que l'on utilise comme produit de départ le composé de formule (X) : 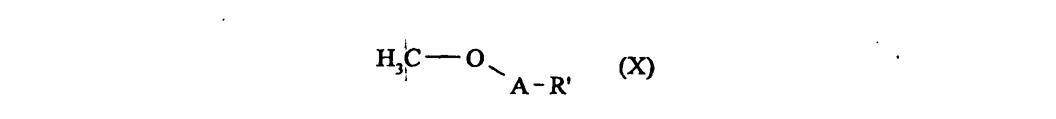 dans laquelle A et R' sont tels que définis précédemment, que l'on soumet à une déméthylation en utilisant des agents classiques comme HBr, AlCl₃, AlBr₃, BBr₃ ou des systèmes binaires acides de Lewis/nucléophiles comme AlCl₃/PhCH₂SH, ou BBr₃/Me₂S par exemple, pour obtenir le composé de formule (XI) :

   HO ― A - R' (XI)

   dans laquelle A et R' sont définis comme précédemment,
   que l'on transforme, grâce à l'action de réactifs tels que POCl₃, PCl₅, Ph₃PBr₂, PhPCl₄, HBr ou HI en dérivé halogéné correspondant de formule (XII) :

   Hal-A-R' (XII)

   dans laquelle A et R' sont définis comme précédemment et Hal représente un atome d'halogène (les composés de formule (XII) pouvant être obtenus par des réactions d'échange comme par exemple le traitement d'un dérivé chloré par KF au sein de la diméthylformamide pour conduire au dérivé fluoré correspondant, ou le traitement d'un dérivé bromé par KI en présence de sels de cuivre pour conduire au dérivé iodé correspondant),
   qui est traité :
   par du monoxyde de carbone et Bu₃SnH, la réaction étant catalysée par du palladium (0), pour conduire à l'aldéhyde correspondant de formule (XIII) : 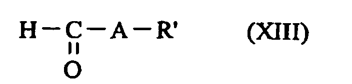 dans laquelle A et R' sont tels que définis précédemment,
   composé de formule (XIII) pouvant par ailleurs être obtenu par des techniques classiques de lithiations à partir du dérivé halogéné de formule (XII), ou par l'intermédiaire du dérivé vinylique correspondant (obtenu à partir du composé de formule (XII) par action de vinyltributylétain et de palladium tetrakis) soumis à une ozonolyse, ou encore par formylation directe du noyau A selon une réaction de Vilsmeier par exemple,
   composé de formule (XIII) que l'on soumet à un agent oxydant pour obtenir le composé de formule (XIV) :

   HOOC―A-R' (XIV)

   dans laquelle A et R' sont définis comme précédemment, qui est :
   transformé, après l'action de chlorure de thionyle et d'un azidure, puis d'un acide, en composé de formule (XV) :

   H₂N―A-R' (XV)

   dans laquelle A et R' sont définis comme précédemment, sur lequel on condense :
   - soit un chlorure d'acyle ClCORₐ ou l'anhydride (mixte ou symétrique) correspondant pour lesquels Rₐ est tel que défini précédemment, pour conduire au composé de formule (I/c), cas particulier des composés de formule (I) : 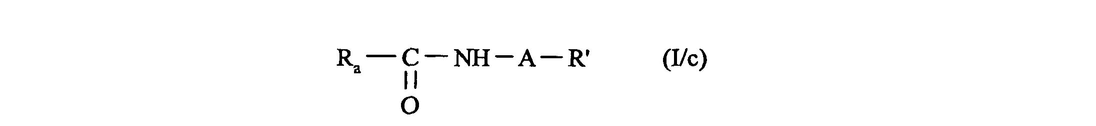 dans laquelle Rₐ, A et R' sont définis comme précédemment,
      qui peut être soumis à l'action d'un composé de formule (XVI) :

      R¹ ₐ―J (XVI)

      dans laquelle R¹ₐ peut prendre toutes les valeurs de Rₐ à l'exception de l'atome d'hydrogène et J représente un groupe partant comme un atome d'halogène ou un groupement tosyle,
      pour obtenir le composé de formule (I/d), cas particulier des composés de formule (I) : 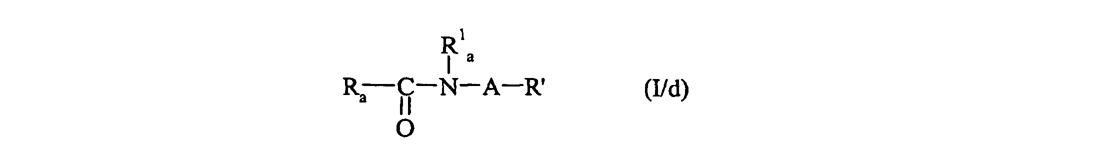 dans laquelle Rₐ, R¹ₐ, A et R' sont tels que définis précédemment,
      les composés de formule (I/c) et (I/d) formant le composé de formule (I/e), cas particulier des composés de formule (I) : 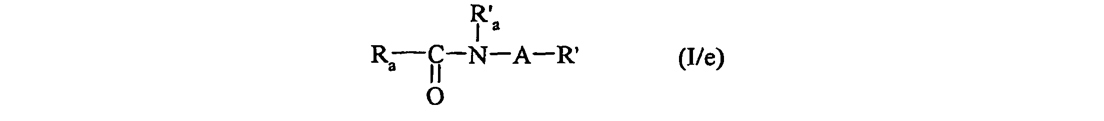 dans laquelle Rₐ, R'ₐ, A et R' ont la même définition que précédemment,
      composé de formule (I/e) que l'on peut soumettre à un agent de thionation tel que le réactif de Lawesson par exemple afin d'obtenir le composé de formule (I/f), cas particulier des composés de formule (I) : 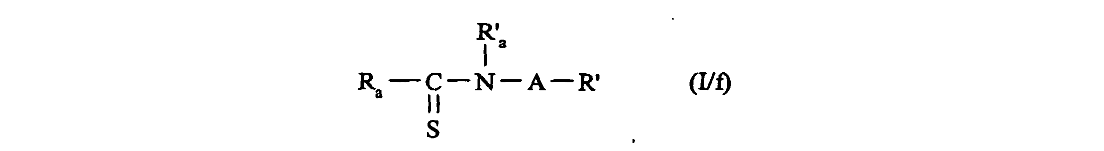 dans laquelle Rₐ, R'ₐ, A et R' sont définis comme précédemment,
   - soit un composé de formule (XVII) :

      Q=C=N-R'ₐ (XVII)

      dans laquelle Q et R'ₐ sont tels que définis précédemment,
      pour conduire au composé de formule (I/g), cas particulier des composés de formule (I) : 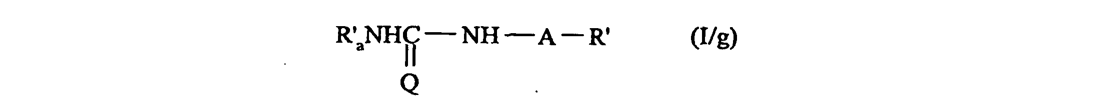 dans laquelle R'ₐ, Q, A et R' sont définis comme précédemment,
      qui peut être soumis à l'action d'un composé de formule (XVI) pour obtenir le composé de formule (I/h), cas particulier des composés de formule (I) : 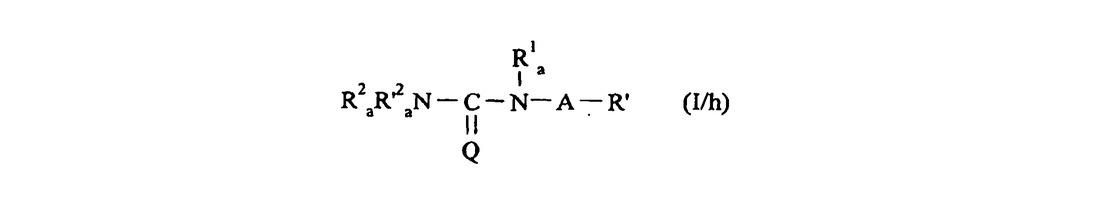 dans laquelle Q, R¹ₐ, A et R' ont la même définition que précédemment et R²ₐ et R'²ₐ, identiques
      ou différents, peuvent prendre toutes les valeurs de Rₐ à l'exception de l'atome d'hydrogène et ne peuvent pas former de structure cyclique avec l'atome d'azote qui les porte,
   - soit un composé de formule (XVIII) : 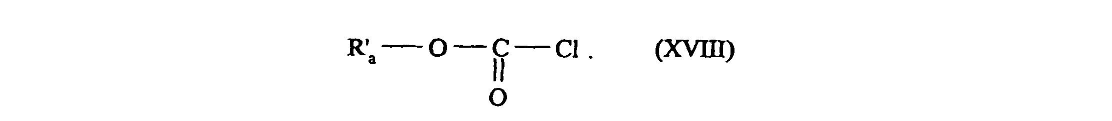 dans laquelle R'ₐ est tel que défini précédemment, ou son anhydride correspondant (R'ₐOCO)₂O,
      pour obtenir le composé de formule (I/i), cas particulier des composés de formule (I) : 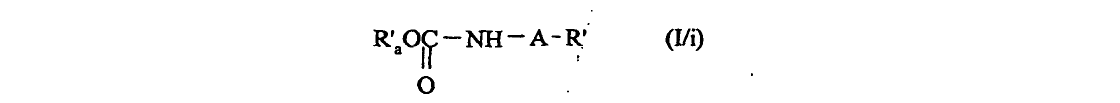 dans laquelle R'ₐ, A et R' sont définis comme précédemment,
      qui peut être soumis à l'action d'un composé de formule (XVI) et/ou à l'action d'un agent de thionation afin de conduire au composé de formule (I/j), cas particulier des composés de formule (I) : 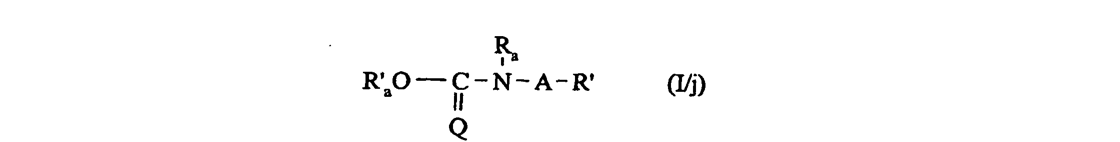 dans laquelle Rₐ, R'ₐ, Q, A et R' sont définis comme précédemment,
   - soit un composé de formule (XIX) :

      RₐSO₂Cl (XIX)

      dans laquelle Rₐ est tel que défini précédemment,
      suivi éventuellement de l'action d'un composé de formule (XVI) pour conduire au composé de formule (I/k), cas particulier des composés de formule (I) : 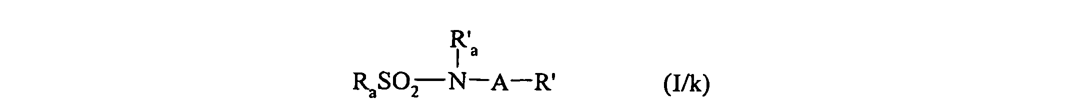 dans laquelle Rₐ, R'ₐ, A et R' sont définis comme précédemment,
      les composés (I/c) à (I/k) pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

Les composés (X) de départ sont soit commerciaux, soit décrits dans la littérature comme par exemple dans les demandes EP0447285, EP0527687, EP0562956, EP0591057, EP0662471, EP0745586, EP0709371, EP0745583, EP0721938, EP0745584, EP0737670, EP0737685, ou WO9738682.

Un autre procédé avantageux de l'invention concernant la préparation des composés de formule (I) est caractérisé en ce que l'on utilise comme produit de départ le composé de formule (XXII): 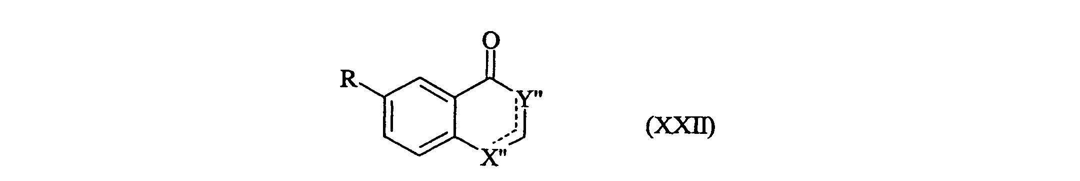 dans laquelle R et la représentation .... sont définis comme dans la formule (I), Y" représente une liaison, et dans ce cas X" représente un atome d'oxygène ou de soufre ou un groupement NR₀ (où R₀ est tel que défini précédemment), ou X" et Y", identiques ou différents, représentent un groupement C(H)q (où q vaut 1 ou 2),
que l'on soumet à une réaction de Wittig puis à une réduction afin de conduire au composé de formule (XXIII) : 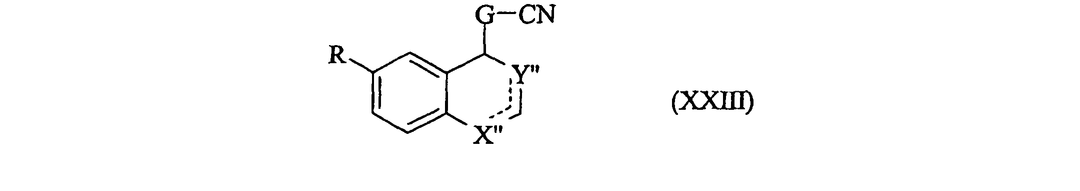 dans laquelle R, X", Y", G et la représentation .... sont définis comme précédemment,
qui peut être oxydé afin de conduire au composé de formule (XXIV) : 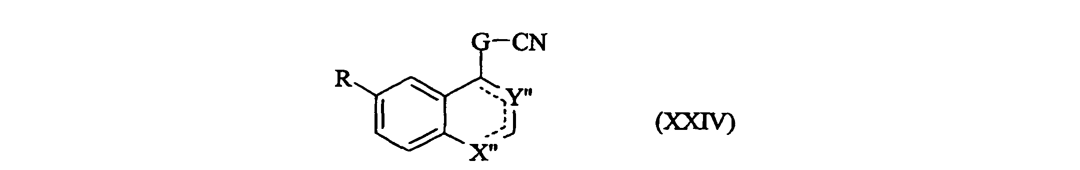 dans laquelle R, X", Y", G et la représentation .... ont la même définition que précédemment, qui est
* soit hydrolysé en milieu acide ou basique puis soumis, après activation sous forme de chlorure d'acide ou en présence d'un agent de couplage, à l'action d'une amine HNR'ₐR"ₐ dans laquelle R'ₐ et R"ₐ sont définis comme précédemment afin de conduire au composé de formule (I/p), cas particulier des composés de formule (I) : 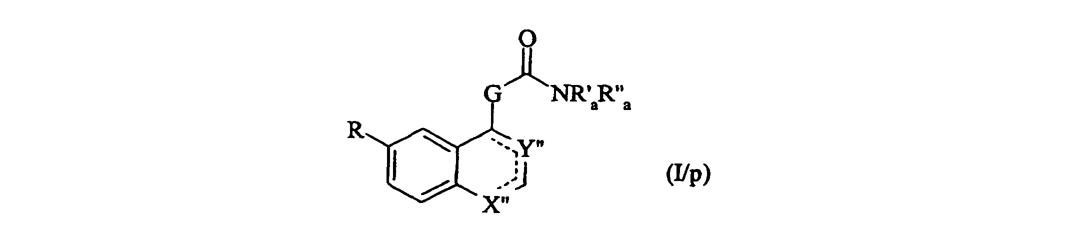 dans laquelle R, X", Y", G, R'ₐ, R"ₐ et la représentation .... sont définis comme précédemment,
   que l'on peut soumettre à un agent de thionation comme le réactif de Lawesson pour conduire au composé de formule (I/q), cas particulier des composés de formule (I) : 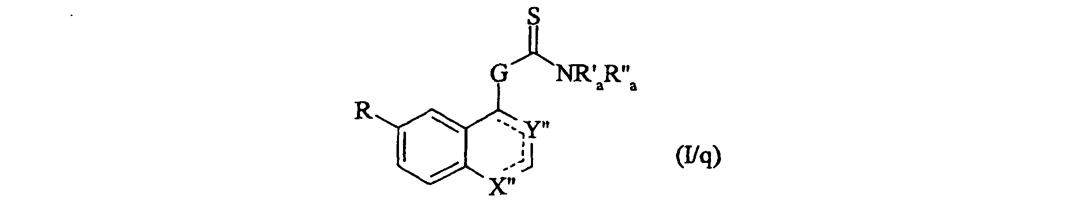 dans laquelle R, X", Y", G, R'ₐ, R"ₐ et la représentation .... sont définis comme précédemment,
* soit hydrolysé en milieu acide ou basique puis transformé en azide correspondant pour conduire, après avoir subi un réarrangement de Curtius et une hydrolyse, au composé de formule (XXV) : 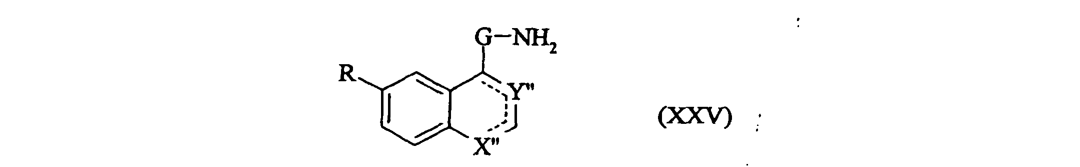 dans laquelle R, X", Y" et G sont tels que définis précédemment,
   qui est mis en réaction avec :
   - un chlorure d'acyle ClCOR'ₐ ou l'anhydride (mixte ou symétrique) correspondant pour lesquels R'ₐ est tel que défini précédemment, suivi éventuellement de l'action d'un composé de formule (XVI) et/ou de l'action d'un agent de thionation afin de conduire au composé de formule (I/r), cas particulier des composés de formule (I) : 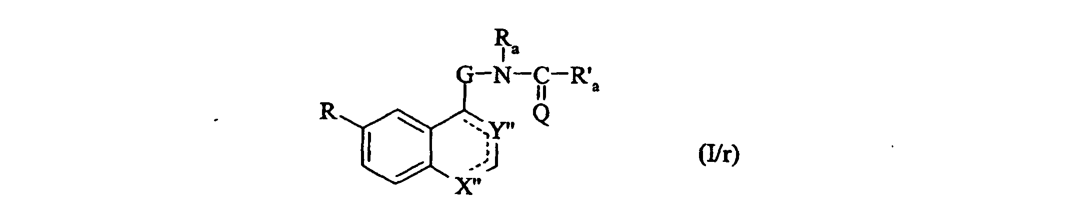 dans laquelle R, X", Y", G, Rₐ, R'ₐ, Q et la représentation .... sont définis comme précédemment,
   - ou avec un composé de formule (XVII) suivi éventuellement de l'action d'un composé de formule (XVI) afin de conduire au composé de formule (I/s), cas particulier des composés de formule (I) : 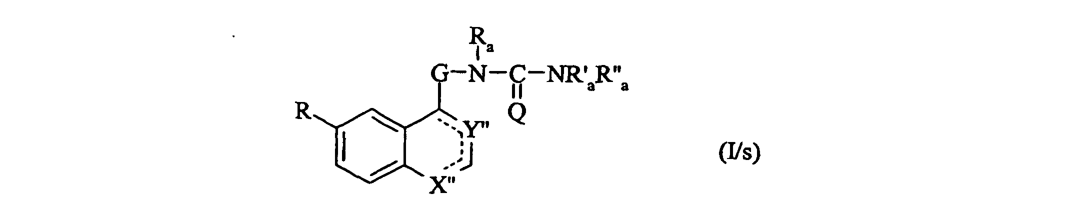 dans laquelle R, X", Y", G, Rₐ, R'ₐ, R"ₐ, Q et la représentation .... sont définis comme précédemment,
      les composés (I/p) à (I/s) pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

Les composés de formule (XXII) sont soit commerciaux, soit aisément accessibles à l'homme du métier
- à partir du composé de formule (XXVI) : 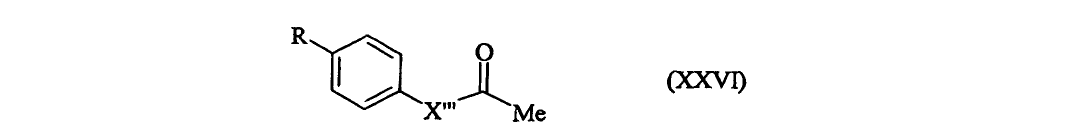 dans laquelle R est tel que défini précédemment, et X"' représente un atome d'oxygène ou de soufre, ou un groupement NR₀ (où R₀ est tel que défini précédemment),
   (le composé de formule (XXVI) étant soit commercial, soit obtenu à partir du composé de formule (XXVI') : 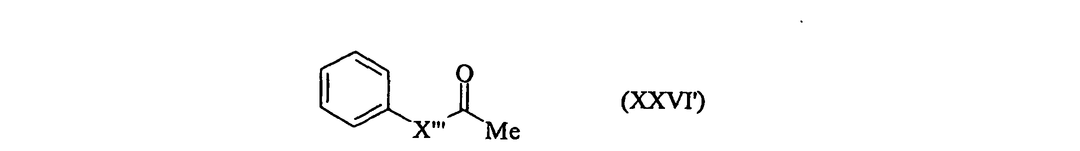 dans laquelle X"' est tel que défini précédemment, par des réactions classiques de substitution du noyau aromatique),
   que l'on soumet à l'action d'AlCl₃, pour conduire au composé de formule (XXVII) : 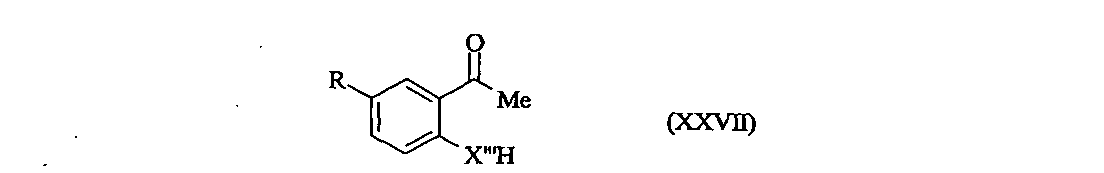 dans laquelle R et X"' sont tels que définis précédemment,
   que l'on soumet à une bromation pour obtenir le composé de formule (XXVIII) : 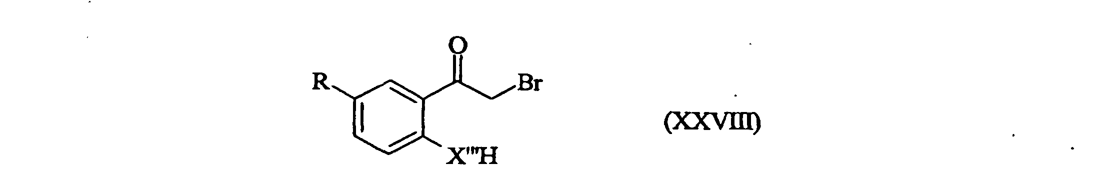 dans laquelle X"' et R sont tels que définis précédemment,
   qui est placé en milieu basique pour conduire au composé de formule (XXIX), cas particulier des composés de formule (XXII) : 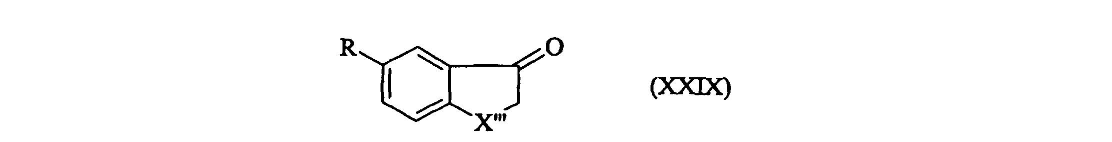 dans laquelle R et X"' sont tels que définis précédemment,
- ou à partir du composé de formule (XXX) : 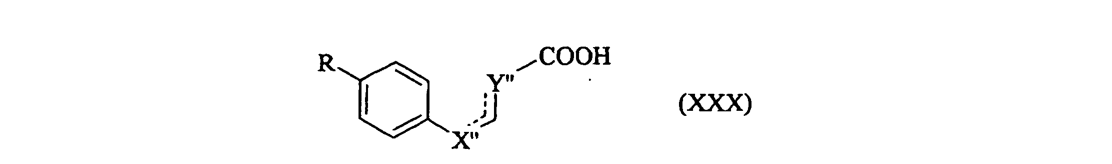 dans laquelle R, X", Y" et la représentation .... ont la même définition que précédemment,
   qui est cyclisé, en présence d'acide polyphosphorique pour conduire au composé de formule (XXII).

Les composés de l'invention et les compositions pharmaceutiques les contenant s'avèrent être utiles pour le traitement des troubles du système mélatoninergique.

L'étude pharmacologique des dérivés de l'invention a en effet montré qu'ils étaient atoxiques, doués d'une haute affinité pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en 1 ou plusieurs prises.

Les exemples suivants illustrent l'invention et ne la limitent en aucune façon. Les préparations suivantes conduisent à des composés de l'invention ou à des intermédiaires de synthèse utiles dans la préparation des composés de l'invention.

### Préuaration 1 : N-[2-(7-Hydroxy-1-naphtyl)éthyl]acétamide

Sous atmosphère inerte, 27,5 mmol de complexe tribromure de bore/diméthylsulfure sont dissoutes dans 100 ml de dichlorométhane et agitées pendant 15 min à température ambiante. Une solution de 13,7 mmol de N-[2-(7-méthoxy-1-naphtyl)éthyl]acétamide dans 50 ml de dichlorométhane est ajoutée, et le milieu réactionnel est porté à reflux pendant 30 heures. Après refroidissement, la réaction est hydrolysée avec précaution et le dichlorométhane est évaporé. Le milieu est alors extrait à l'acétate d'éthyle, les phases organiques regroupées sont lavées par une solution aqueuse de bicarbonate de potassium 1M, puis par une solution de soude 1M. La phase organique est séchée sur sulfate de magnésium, et concentrée pour conduire au composé du titre.

### Préparation 2 : N-[2-(7-Hydroxy-1-naphtyl)éthyl]-2-phénylacétamide

### Préparation 2 : N-[2-(7-Hydroxy-1-naphtyl)éthyl]-2-phénylacétamide

On procède comme dans la Préparation 1, mais en remplaçant le N-[2-(7-méthoxy-1-naphtyl) éthyl]acétamide par le N-[2-(7-méthoxy-1-naphtyl)éthyl]-2-phénylacétamide.

Dans les préparations 3 à 37, on procède comme dans la préparation 1, mais en remplaçant le N-[2-(7-méthoxy-1-naphtyl)éthyl]acétamide par le substrat de départ méthoxylé approprié.

### Préparation 3 : N-[2-(7-Hydroxy-1-naphtyl)éthyl]-2-bromoacétamide

### Préparation 6 : N-Cyclohexyl-4-(7-hydroxy-1-naphtyl)butanamide

### Préparation 8 : N-[3-(7-Hydroxy-1-naphtyl)propyl]acétamide

### Préparation 13 : N-[2-(7-Hydroxy-3-phényl-1-naphtyl)éthyl]acétamide

### Préparation 14 : N-[2-(3-Benzoyl-7-hydroxy-1-naphtyl)éthyl]-N'-propylurée

### Préparation 15 : N-{2-[3-(Cyclopropylméthyl)-7-hydroxy-1-naphtyl]éthyl}acétamide

### Préparation 17 : N-Méthyl-4-(5-Hydroxybenzo[b]furan-3-yl)butanamide

### Préparation 18 : N-[2-(5-Hydroxybenzo[b]furan-3-yl)éthyl]acétamide

### Préparation 21 : N-[2-(5-Hydroxy-1H-3-indolyl)éthyl]benzamide

### Préparation 23 : N-[2-(2-Benzyl-5-hydroxybenzo[b]furan-3-yl)éthyl]-1-cyclopropane carboxamide

### Préparation 35 : N-{2-[7-Hydroxy-3-naphtyl-1-naphtyl]éthyl}heptanamide

### Préparation 36 : N-[2-(7-Hydroxy-1,2,3,4-tétrahydro-1-naphtalényl)éthyl]acétamide

### Préparation 38 : N-Cyclohexyl-4-(7-chloro-1-naphtyl)butanamide

On fait buller du chlore (10 mmol) dans la dichlorophénylphosphine à un flux tel que la température de réaction est maintenue entre 70 et 80°C. Après addition complète du chlore, le tétrachlorure de phénylphosphine ainsi obtenu est un liquide jaune pâle. On ajoute 10 mmol du produit obtenu dans la Préparation 6 en une seule portion, et le mélange réactionnel est chauffé à 160°C pendant la nuit. Après refroidissement, la solution est versée dans un mélange eau/glace (20 ml) et neutralisée avec une solution aqueuse d'hydroxyde de sodium à 50 %. Après extraction à l'éther, les phases organiques sont séchées et concentrées sous pression réduite pour donner un résidu qui est chromatographié sur gel de silice afin d'obtenir le produit du titre pur.

### Préparation 40 : N-[2-(7-Bromo-1-naphtyl)éthyl]-2-phénylacétamide

Dans un tricol de 150 ml équipé d'une ampoule à brome, d'un réfrigérant surmonté d'un tube rempli de chlorure de calcium, et d'un agitateur mécanique, on verse de la triphénylphosphine (10 mmol) et de l'acétonitrile (70 ml). La solution est refroidie à l'aide d'un bain de glace en maintenant l'agitation et on additionne le brome (10 mmol). A la fin de l'addition, le bain de glace est retiré puis on ajoute le produit obtenu dans la Préparation 2 (8 mmol). Le mélange réactionnel est agité à 60-70°C jusqu'à disparition du produit de départ (suivi par CCM). En fin de réaction, le mélange est filtré puis le filtrat est concentré sous pression réduite. Le résidu est repris dans l'acétate d'éthyle, lavé à l'eau puis avec une solution saturée d'hydrogénocarbonate de potassium, et encore une fois à l'eau puis séché sur sulfate de magnésium et concentré sous pression réduite. Le résidu est filtré sur gel de silice pour conduire au produit du titre.

Dans les préparations 41 à 72.1, on procède comme dans la Préparation 40 en partant du réactif approprié.

### Préparation 45 : N-[2-(3-Benzoyl-7-bromo-1-naphtyl)éthyl]-N'-propylurée

*Produit de départ : Préparation 14*

### Préparation 57 : N-[2-(7-Bromo-3-naphtyl-1-naphtyl)éthyl]heptanamide

*Produit de départ : Préparation 35*

### Préparation 58 : N-[2-(7-Bromo-1-naphtyl)éthyl]acétamide

*Produit de départ : Préparation 1*

### Préparation 60: N-[2-(7-Bromo-1-naphtyl)éthyl]-2-bromoacétamide

*Produit de départ : Préparation 3*

### Préparation 62 : N-[3-(7-Bromo-1-naphtyl)propyl]acétamide

*Produit de départ : Préparation 8*

### Préparation 64 : N-{2-[7-Bromo-3-(cyclopropylméthyl)1-naphtyl]éthyl}acétamide

*Produit de départ : Préparation 15*

### Préparation 65 : N-Méthyl-3-(5-bromobenzo[b]furan-3-yl)butanamide

*Produit de départ : Préparation 17*

### Préparation 67 : N-[2-(5-Bromo-1H-3-indolyl)éthyl]benzamide

*Produit de départ : Préparation 21*

### Préparation 68 : N-[2-(2-Benzyl-5-bromobenzo[b]furan-3-yl)éthyl]-1-cyclopropane carboxamide

*Produit de départ : Préparation 23*

### Préparation 71: N-[2-(7-Bromo-3-phényl-1-naphtyl)éthyl]acétamide

*Produit de départ : Préparation 13*

### Préparation 72 : N-[2-(5-Bromobenzo[b]furan-3-yl)éthyl]acétamide

*Produit de départ : Préparation 18*

### Préparation 72.1 : N-[2-7-Bromo-1,2,3,4-tétrahydro-1-naphtalényl)éthyl]acétamide

*Produit de départ : Préparation 36*

### Préparation 73 : N-[2-(7-Iodo-1-naphtyl)éthyl]-2-phénylacétamide

Un mélange du produit obtenu dans la Préparation 40 (2 mmol), d'iodure de potassium (30 mmol) et d'iodure de cuivre 1 (10 mmol) dans l'hexaméthyl phosphoramide (6 ml) est chauffé à 150-160°C avec agitation sous atmosphère d'azote jusqu'à ce qu'un taux de conversion de 90 % soit atteint (suivi en CCM). On ajoute alors de l'acide chlorhydrique dilué puis de l'éther et la mixture est alors filtrée pour éliminer les sels de cuivre (I) insolubles. La phase organique est séparée, lavée avec une solution de sulfite de sodium, de l'eau, séchée sur sulfate de magnésium et évaporée pour donner un résidu que l'on chromatographie sur gel de silice pour conduire au produit du titre.

Dans les préparations 74 à 108, on procède comme dans la Préparation 73, mais en remplaçant le produit de la Préparation 40 par le substrat approprié.

### Préparation 78: N-[2-(3-Benzoyl-7-iodo-1-naphtyl)éthyl]-N'-propylurée

*Produit de départ : Préparation 45*

### Préparation 91 : N-(2-(7-Iodo-3-naphtyl-1-naphtyl)éthyl]heptanamide

*Produit de départ : Préparation 57*

### Préparation 92 : N-[2-(7-Iodo-1-naphtyl)éthyl]acétamide

*Produit de départ : Préparation 58*

### Préparation 94 : N-[2-(7-Iodo-1-naphtyl)éthyl]-2-bromoacétamide

*Produit de départ : Préparation 60*

### Préparation 96 : N-Cyclohexyl-4-(7-Iodo-1-naphtyl)butanamide

*Produit de départ : Préparation 38*

### Préparation 97 : N-[3-(7-Iodo-1-naphtyl)propyl]acétamide

*Produit de départ : Préparation 62*

### Préparation 99 : N-{2-[7-Iodo-3-(cyclopropylméthyl)-1-naphtyl]éthyl}acétamide

*Produit de départ : Préparation 64*

### Préparation 100 : N-Méthyl-4-(5-iodobenzo[b]furan-3-yl)butanamide

*Produit de départ : Préparation 65*

### Préparation 102 : N-[2-(5-Iodo-1H-3-indolyl)éthyl]benzamide

*Produit de départ : Préparation 67*

### Préparation 103 : N-[2-(2-Benzyl-5-iodobenzo[b]furan-3-yl)éthyl]-1-cyclopropane carboxamide

*Produit de départ: Préparation 68*

### Préparation 106 : N-[2-(7-Iodo-3-phényl-1-naphtyl)éthyl]acétamide

*Produit de départ : Préparation 71*

### Préparation 107 : N-[2-(7-Iodo-1,2,3,4-tétrahydro-1-naphtalényl)éthyl]acétamide

*Produit de départ : Préparation 72.1*

### Préparation 108 : N-[2-(5-Iodobenzo[b]furan-3-yl)éthyl]acétamide

*Produit de départ : Préparation 72*

### Préparation 109 : N-[2-(7-Amino-1-naphtyl)éthyl]-2-phénylacétamide

### Stade A : N-[2-(7-Vinyl-1-naphtyl)éthyl]-2-phénylacétamide

15 mmol du produit obtenu dans la Préparation 73, 16 mmol de vinyl tributylétain et 0,43 mmol de (triphénylphosphine) palladium tetrakis, sont portés sous agitation à 110°C pendant 3 heures dans 30 ml de N-méthylpyrrolidinone. Après évaporation du solvant, le résidu est repris dans 20 mL de dichlorométhane et traité par une solution aqueuse 10 % de fluorure de potassium. Après extraction, concentration sous pression réduite et chromatographie sur gel de silice, on obtient le produit du titre pur.

### Stade B : N-[2-(7-Formyl-1-naphtyl)éthyl]-2-phénylacétamide

A une solution de 10 mmol du produit obtenu dans le stade A dans un mélange de 50 ml de dioxane et 25 ml d'eau est ajouté à température ambiante 1,10 g de tétroxyde d'osmium dans le 2-méthyl-2-propanol, puis 8,70 g de Periodate de sodium. Après agitation une nuit à température ambiante, la suspension est filtrée, le filtrat concentré sous pression réduite. Le résidu obtenu est repris dans le dichlorométhane. La phase organique est lavée avec de l'eau, séchée et évaporée. Le résidu est purifié par chromatographie sur gel de silice pour conduire au produit du titre.

### Stade C : Acide 8-{2-[(2-Phénylacétyl)amino}éthyl}-2-naphtoïque

A une solution de 6,88 mmol du produit obtenu dans le stade B dans 30 ml d'acétone sont ajoutés à température ambiante 2,7 g de permanganate de potassium dans 50 ml d'un mélange acétone/eau (50/50). La solution est agitée 2 heures à température ambiante puis filtrée. Le filtrat est concentré sous pression réduite et chromatographié sur gel de silice pour conduire au produit du titre.

### Stade D : Chlorure de 8-{2-[(2-phénylacétyl)amino]éthyl}-2-napthtalènecarbonyle

5 mmol du produit obtenu dans le stade C sont dissoutes dans 40 ml de chlorure de thionyle. Après agitation sous atmosphère inerte pendant 1 heure, le chlorure de thionyle est évaporé sous pression réduite pour conduire au produit du titre.

### Stade E : N-[2-(7-Amino-1-naphtyl)éthyl]-2-phénylacétamide

Une solution du produit obtenu dans le stade D (20 mmol) dans le dichlorométhane (30 ml) contenant du bromure de tétrabutyl ammonium (20 mg) est refroidie dans un bain de glace. Après addition de l'azoture de sodium (24 mmol) dissous dans 5 ml d'eau, la solution est agitée vigoureusement à 0°C pendant 2 heures. La phase organique est séparée, lavée à l'eau (2 x 5 ml) et séchée sur sulfate de magnésium. Après filtration, on ajoute l'acide trifluoroacétique (30 mmol) et la solution est agitée sous reflux pendant 60 heures. Après refroidissement, la phase organique est lavée avec une solution saturée d'hydrogénocarbonate de sodium (2 x 5 ml) et concentrée sous pression réduite. Le résidu est alors repris dans le méthanol (20 ml) et on ajoute de l'eau (80 ml) puis du carbonate de potassium (30 mmol). Après agitation à température ambiante pendant 20 heures, le milieu réactionnel est concentré sous pression réduite jusqu'à un volume de 60 ml environ puis extrait 3 fois à l'éther (3 x 50 ml). Après séchage sur sulfate de sodium, la phase organique est filtrée puis évaporée sous pression réduite. Le résidu est chromatographié sur gel de silice pour conduire au produit du titre.

Dans les Préparations 110 à 134, on procède comme dans l'exemple 109 en partant du substrat approprié.

### Préparation 110 : N-[2-(7-Amino-1-naphtyl)éthyl]-2-bromoacétamide

*Produit de départ : Préparation 94*

### Préparation 112 : N-Cyclohexyl-4-(7-amino-1-naphtyl)butanamide

*Produit de départ : Préparation 96*

### Préparation 113 : N-[3-(7-Amino-1-naphtyl)propyl]acétamide

*Produit de départ : Préparation 97*

### Préparation 115 : N-[2-(7-Amino-3-benzoyl-1-naphtyl)éthyl]-N'-propylurée

*Produit de départ : Préparation 78*

### Préparation 117 : N-[2-(7-Amino-1-naphtyl)éthyl]acétamide

*Produit de départ : Préparation 92*

### Préparation 118 : N-{2-[7-Amino-3-(cyclopropylméthyl)-1-naphtyl]éthyl}acétamide

*Produit de départ : Préparation 99*

### Préparation 119 : N-Méthyl-4-(5-aminobenzo[b]furan-3-yl)butanamide

*Produit de départ : Préparation 100 .*

### Préparation 121 : N-[2-(5-Amino-1H-3-indolyl)éthyl]benzamide

*Produit de départ : Préparation 102*

### Préparation 123 : N-[2-(5-Amino-2-benzylbenzo[b]furan-3-yl)éthyl]-1-cyclopropane carboxamide

*Produit de départ : Préparation 103*

### Préparation 129 : N-[2-(7-Amino-3-phényl-1-naphtyl)éthyl]acétamide

*Produit de départ : Préparation 106*

### Préparation 131 : N-[2-(7-Amino-3-naphtyl-1-naphtyl)éthyl]heptanamide

*Produit de départ : Préparation 91*

### Préparation 132 : N-[2-(5-Aminobenzo[b]furan-3-yl)éthyl]acétamide

*Produit de départ : Préparation 108*

### Préparation 133 : N-[2-(7-Amino-1,2,3,4-tetrahydro-1-naphtalényl)éthyl]acétamide

*Produit de départ : Préparation 107*

Les Préparations 135 à 145 sont obtenues en procédant comme dans la Préparation 1 à partir du substrat approprié.

### Préparation 140 : N-[2-(5-Hydroxy-1-benzofuran-3-yl)éthyl]cyclopropane carboxamide

### Préparation 166 : N-[2-(5-Bromo-1-benzofuran-3-yl)éthyl]cyclopropane carboxamide

On procède comme dans la Préparation 40 à partir du composé obtenu dans la Préparation 140.

### Préparation 167 : N-[2-(5-Iodo-1-benzofuran-3-yl)éthyl]cyclopropane carboxamide

On procède comme dans la Préparation 73 à partir du composé obtenu dans la Préparation 166.

### Préparation 168 : N-[2-(5-Amino-1-benzofuran-3-yl)éthyl]cyclopropane carboxamide

On procède comme dans la Préparation 109 à partir du composé obtenu dans la Préparation 167.

### Préparation 169 : N-[2-(5-Hydroxy-1-benzothiophèn-3-yl)éthyl]benzamide

On procède comme dans la Préparation 1.

### Préparation 170 : N-[2-(5-Bromo-1-benzothiophèn-3-yl)éthyl]benzamide

On procède comme dans la Préparation 40 à partir du composé obtenu dans la Préparation 169.

### Préparation 171 : N-[2-(5-Iodo-1-benzothiophèn-3-yl)éthyl]benzamide

On procède comme dans la Préparation 73 à partir du composé obtenu dans la Préparation 170.

### Préparation 172 : N-[2-(5-Amino-1-benzothiophèn-3-yl)éthyl]benzamide

On procède comme dans la Préparation 109 à partir du composé obtenu dans la Préparation 171.

### EXEMPLE 1 : N-{8-[2-([2-Phénylacétyl]amino)éthyl]-2-naphtyl}butanamide

A une solution du produit obtenu dans la Préparation 109 (10 mmol) dans l'éther (10 ml) et la triéthylamine (2 ml), on ajoute goutte à goutte une solution de chlorure d'acide butanoïque (11 mmol) en solution dans l'éther (5 ml). On agite la solution à température ambiante jusqu'à disparition de l'amine (suivi par CCM). En fin de réaction, la phase organique est lavée à l'eau, séchée, concentrée sous pression réduite et chromatographiée sur gel de silice pour donner le produit du titre.

### EXEMPLE 2 : N-{2-[7-{[(Cyclohexylamino)carbonyl]amino}-1-naphtyl]éthyl}-2-phénylacétamide

A une solution du produit obtenu dans la Préparation 109 (10 mmol) dans le dichlorométhane (10 ml), on ajoute goutte à goutte une solution d'isocyanate de cyclohexyle dans le dichlorométhane (5 ml). On agite à température ambiante jusqu'à disparition de l'amine de départ (suivi par CCM) puis le mélange réactionnel est évaporé et concentré sous pression réduite puis chromatographié sur gel de silice pour conduire au produit du titre.

### EXEMPLE 3 : N-{2-[7-([Anilinocarbothioyl]amino)-1-naphtyl]éthyl}-2-phénylacétamide

On procède comme dans l'Exemple 2 mais en remplaçant l'isocyanate de cyclohexyle par l'isothiocyanate de phényle pour obtenir le produit du titre.

Dans les exemples 4 à 16, on procède comme dans l'Exemple 1 à partir des réactifs appropriés.

### EXEMPLE 4 : N-(8-{2-[(2-bromoacétyl)amino]éthyl}-2-naphtyl)-1-cyclohexane carboxamide

*Produit de départ : Préparation 110*

### EXEMPLE 6 : N-{6-Benzoyl-8-[2-{[{propylamino)carbonyl]amino}éthyl]-2-naphtyl}-2,2-diméthylpropanamide

*Produit de départ : Préparation 115*

### EXEMPLE 7 : N-{3-(4-(Méthylamino)-4-oxobutyl]benzo[b]furan-5-yl}-3-butynamide

*Produit de départ : Préparation 119*

### EXEMPLE 13 : N-{8-[2-(Acétylamino)éthyl]-6-phényl-2-naphtyl}-1-cyclopentane carboxamide

*Produit de départ : Préparation 129*

### EXEMPLE 15 : N-{8-[2-(Heptanoylamino)éthyl]-2,6-dinaphtyl}-2-butènamide

*Produit de départ : Préparation 131*

Les exemples 17 à 23, sont obtenus en procédant comme dans l'Exemple 2 à partir des réactifs appropriés.

### EXEMPLE 17 : N-Cyclohexyl-4-{7-[(anilinocarbonyl)amino]-1-naphtyl}butanamide

*Produit de départ : Préparation 112*

### EXEMPLE 19 : N-{2-[7-{[(benzylamino)carbonyl]amino}-3-(cyclopropylméthyl)-1-naphtyl]éthyl}acétamide

*Produit de départ : Préparation 118*

### EXEMPLE 21 : N-{2-[2-Benzyl-5-([(1-éthynylamino)carbonyl]amino}benzo[b]furan-3-yl] éthyl}-1-cyclopropanecarboxamide

*Produit de départ : Préparation 123*

### EXEMPLE 23 : N-12-(7-{[(Cyclohexylamino)carbonyl]amino}-3-phényl-1-naphtyl)éthyl] acétamide

*Produit de départ : Préparation 129*

Dans les exemples 24 à 29, on procède comme dans l'Exemple 3 à partir des substrats appropriés.

### EXEMPLE 24 : N-{2-[7-{[(Isobutylamino)carbothioyl]amino}-1-naphtyl]éthyl}-2-bromo acétamide

*Produit de départ : Préparation 110*

### EXEMPLE 25 : N-{3-[7-{[([4-Méthylbenzyl]amino)carbothioyl]amino}-1-naphtyl] propyl}acétamide

*Produit de départ : Préparation 113*

### EXEMPLE 26 : N-Méthyl-4-{5-[([1-éthynylamino]carbothioyl)amino]benzo[b]furan-3-yl} butanamide

*Produit de départ : Préparation 119*

### EXEMPLE 27 : N-{2-[5-{[(Butylamino)carbothioyl]amino}-1H-3-indoyl]éthyl}benzamide

*Produit de départ : Préparation 121*

Dans les Exemples 58 à 61, on procède comme dans l'Exemple 1 en remplaçant le chlorure d'acide par l'halogéno carboxylate correspondant.

### EXEMPLE 58 : N-{3-(2-(Acétylamino)éthyl]benzo[b]furan-5-yl}carbamate de méthyle

*Produit de départ : Préparation 132*

*Point de fusion* =*138-140°C*

### EXEMPLE 59 : N-(8-{2-[(2-Bromoacétyl)amino]éthyl}-2-naphtyl)carbamate d'éthyle

*Produit de départ : Préparation 110*

### EXEMPLE 60 : N-{8-[2-(Acétylamino)éthyl]-6-phényl-2-naphtyl}carbamate de méthyle

*Produit de départ : Préparation 129*

### EXEMPLE 61 : N-{8-[2-(Acétylamino)éthyl]-5,6,7,8-tétrahydro-2-naphtalényl} carbamate d'hexyle

*Produit de départ : Préparation 133*

### EXEMPLE 62 : N-[2-(5-Méthoxycarbonylbenzo[b]furan-3-yl)éthyl]acétamide

*Point de fusion : 121-122°C*

### EXEMPLE 74 : N-{3-[2-(Acétylamino)éthyl]-1-benzofuran-5-yl}-2,2,2-trifluoroacétamide

### Stade A : Acide 3-[2-(Acétylamino)éthyl]-1-benzofuran-5-carboxylique

A l'ester obtenu dans l'Exemple 62 (2 mmol) dissous dans du méthanol (90 ml), on ajoute une solution aqueuse de soude à 30 % (30 ml) et le milieu est agité pendant une nuit. Après évaporation du méthanol, la température du milieu réactionnel est abaissée à l'aide d'un bain de glace et on acidifie avec une solution d'acide chlorhydrique (6N). La phase aqueuse est extraite par de l'acétate d'éthyle, la phase organique séchée sur du sulfate de magnésium puis évaporée à sec. Le résidu obtenu est recristallisé.
*Point de fusion = 210-211°C*

### Stade B : 3-[2-(Acétylamino)éthyl]-1-benzofuran-5-carbonyl azide

L'acide (1 mmol) obtenu au stade A est dissous dans l'acétone. La température du milieu réactionnel est abaissée à l'aide d'un bain de glace et on ajoute la triéthylamine (1,5 mmol) puis le chloroformiate d'éthyle (1,5 mmol). Après 15 minutes d'agitation, on ajoute l'azidure de sodium (1,5 mmol) préalablement dissous dans de l'eau (1 ml d'eau pour 400 mg d'azidure de sodium) puis on laisse à nouveau sous agitation pendant 10 minutes. Le milieu est extrait à l'acétate d'éthyle, puis la phase organique est lavée à l'eau, séchée sur du sulfate de magnésium et évaporée à sec. L'azide obtenu est engagé sans purification supplémentaire, dans l'étape suivante.

### Stade C : N-{3-[2-(Acétylamino)éthyl]-1-benzofuran-5-yl}-2,2,2-trifluoroacétamide

A l'azide obtenu au stade B (460 mg, 1,68 mmol) dissous dans du dichlorométhane, on ajoute l'acide trifluoroacétique (1,82 ml, 2,35 mmol) et on laisse sous agitation pendant une nuit. Le milieu réactionnel est lavé à l'eau, puis avec une solution aqueuse d'hydrogénocarbonate de sodium à 10 %. La phase organique est séchée sur du sulfate de magnésium, puis évaporée. Le résidu obtenu est purifié par chromatographie sur colonne de gel de silice avec pour éluant, l'acétate d'éthyle.
*Point de fusion* = *152-154°C*

### EXEMPLE 75 : 3-(2-[(Cyclopropylcarbonyl)amino]éthyl}-1-benzofuran-5-yl-carbarmate de méthyle

L'azide obtenu au stade B de l'Exemple 74 (1 mmol) est chauffé à 80°C pendant 1 nuit dans 5 ml de MeOH et 5 ml de toluène. Après évaporation du solvant, le résidu obtenu est purifié par chromatographie sur colonne de gel de silice.
*Point de fusion* = *153-155°C*
*Microanalyse élémentaire :*

| | *C* | *H* | *N* |
|---|---|---|---|
| *% Calculé* | *63,56* | *6,00* | *9,27* |
| *% Trouvé* | *63, 27* | *6, 02* | *9,14* |

### EXEMPLE 76 : 3-[2-(Acétylamino)éthyl]-1-benzofuran-5-yl-carbamate de tert-butyle

On procède comme dans les Exemples 74 et 75.
*Point de fusion* = *146-148°C*
*Microanalyse élémentaire :*

| | *C* | *H* | *N* |
|---|---|---|---|
| *% Calculé* | *64,12* | *6,97* | *8,79* |
| *% Trouvé* | *64, 03* | *6,58* | *8, 67* |

### EXEMPLE 77 : 3-[2-(Acétylamino)éthyl]-1-benzofuran-5-yl-(méthyl)carbamate de tert-butyle

A 1,98 mmole du carbamate obtenu dans l'Exemple 76 dissoute dans du diméthylformamide, on ajoute à froid 2,18 mmol d'hydrure de sodium et on laisse sous agitation pendant 2 heures, à température ambiante. On ajoute au milieu 2,37 mmol d'iodure de méthyle et on laisse agiter pendant 4 heures à température ambiante. Le milieu réactionnel est hydrolysé, extrait à l'acétate d'éthyle, puis la phase organique est lavée à l'eau, séchée sur du sulfate de magnésium. Le résidu est recristallisé.

### EXEMPLE 78 : 3-[2-(Benzoylamino)éthyl]-1-benzofuran-5-yl carbamate de méthyle

On procède comme dans les Exemples 74 et 75.

### EXEMPLE 79 : 3-[2-(Isobutyrylamino)éthyl]-1-benzofuran-5-yl carbamate de méthyle

On procède comme dans les Exemples 74 et 75.

Les exemples 106 à 108 sont obtenus en procédant comme dans l'Exemple 1 à partir du substrat approprié.

### EXEMPLE 106: N-{8-[2-(Acétylamino)éthyl]-2-naphtyl}acétamide

*Produit de départ : Préparation 117*

### EXEMPLE 107: N-{2-[5-(Acétylamino)-1-benzofuran-3-yl]éthyl}cyclopropane carboxamide

*Produit de départ : Préparation 168*

### EXEMPLE 108: N-{2-[5-(Acétylamino)-1-benzothiophèn-3-yl]éthyl}benzamide

*Produit de départ : Préparation 172*

### EXEMPLE 113 : N-{8-[2-(Acétylamino)éthyl]-5,6,7,8-tétrahydro-2-naphtalényl}acétamide

### Stade A : 7-Nitro-3,4-dihydro-1(2H)-naphtalénone

7 mmoles de 1-oxo-1,2,3,4-tétrahydronaphtalène et 5 ml d'acide sulfurique concentrés sont refroidis au congélateur pendant 30 minutes. Le milieu réactionnel est ensuite placé dans un bain d'alcool à -15°C sur une plaque réfrigérante. Le mélange sulfonitrique (1,1 ml d'acide sulfurique et 0,73 ml d'acide nitrique) est préparé et amené à la température du milieu réactionnel avant d'être ajouté goutte à goutte en évitant tout échauffement brutal du milieu. On laisse agiter 15 minutes puis une hydrolyse sur glace pilée est réalisée. Le précipité jaune pâle obtenu est lavé à l'eau jusqu'à obtention d'un pH neutre des eaux de lavage puis séché au dessicateur et purifié par chromatographie sur gel de silice.
*Point defusion* = *103,7-104,3°C*

### Stade B : 2-[7-Nitro-3,4-dihydro-1(2H)-naphtalénylidène]acétonitrile

Dans un bicol de 50 ml sous courant d'azote placé dans un bain d'alcool à ―15°C, on ajoute sous agitation magnétique 0,32 g d'hydrure de sodium par portions dans 40 ml de THF, puis goutte à goutte 1,4 g de cyanométhylphosphonate de diéthyle dans 10 ml de THF. Après une demi-heure d'agitation, lorsque le milieu est bien homogène, on plonge le ballon dans un milieu à -78°C (cryostat), et on ajoute goutte à goutte 1 g du composé obtenu au stade A dissous dans 20 ml de THF. On poursuit l'agitation pendant 2 heures sous courant d'azote. Le milieu réactionnel est ensuite ramené à température ambiante, hydrolysé sur la glace et laissé précipiter. Après essorage, lavage à l'eau jusqu'à pH neutre des eaux de lavage, on extrait par 3 volumes d'éther. Les phases organiques sont lavées par 3 volumes d'eau, puis séchées. Le solide gris obtenu est décoloré sur charbon.
*Point de fusion* = *98,1-98,5°C*

### Stade C: N-{8-[2-(Acétylamino)éthyl]-5,6,7,8-tétrahydro-2-naphtalényl}acétamide

9 mmol du composé obtenu au stade B sont dissoutes dans de l'anhydride acétique (100 ml) puis une petite spatule d'acétate de sodium est ajoutée. Le mélange est introduit dans l'autoclave, on ajoute le nickel de Raney, et on autoclave sous pression à 40 bars, sous agitation à 50-60°C, pendant 6 heures. Le milieu est filtré, rincé à l'alcool à 95°, puis le solvant est évaporé. On hydrolyse par 100 ml d'eau distillée et on extrait par 3 volumes de dichlorométhane. La phase organique est lavée par 2 volumes d'eau, séchée sur sulfate de magnésium, filtrée et évaporée.
On rince par un mélange éther/dichlorométhane, et on triture dans l'éther. Le solide beige clair obtenu est recristallisé.
*Point de fusion* = *127, 7-128, 7°C*
*Microanalyse élémentaire :*

| | *C* | *H* | *N* |
|---|---|---|---|
| *% Calculé* | *70, 04* | *8, 08* | *10, 21* |
| *% Trouvé* | *69, 74* | *8,14* | *10,43* |

### EXEMPLE 114 : 8-[2-(Acétylamino)éthyl]-2-naphtyl carbamate de méthyle

On procède comme dans l'Exemple 1 en remplaçant le chlorure d'acide par l'halogénocarboxylate correspondant.

*Produit de départ : Préparation 117*

### EXEMPLE 146 : 3-[2-(2-Furoylamino)éthyl]-1-benzofuran-5-yl carbamate de méthyle

On procède comme dans les Exemples 74 et 75.

### EXEMPLE 147 : 3-{2-[(Cyclopentylcarbonyl)amino]éthyl}-1-benzofuran-5-yl carbamate de méthyle

On procède comme dans les Exemples 74 et 75.

### EXEMPLE 151 : 8-[2-(3-Buténoylamino)éthyl]-2-naphtyl carbamate de méthyle

On procède comme dans les Exemples 74 et 75.

### EXEMPLE 152 : N-{8-[2-(Acétylamino)éthyl)-2-naphtyl}-4-fluorbenzamide

On procède comme dans l'Exemple 1 à partir du composé obtenu dans la Préparation 117.

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal ofNeuroendocrinology, 1, pp. 1-4, 1989).

### Protocole

1) Les membranes de pars tuberalis de mouton sont préparées et utilisées comme tissu cible dans des expériences de saturation pour déterminer les capacités et affinités de liaison pour la 2-[¹²⁵I]-iodomélatonine.
2) Les membranes de pars tuberalis de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : 1. Etude de liaison aux récepteurs mt₁ et MT₂ de la mélatonine

Les expériences de liaison aux récepteurs mt₁ ou MT₂ sont réalisées en utilisant la 2-[¹²⁵I]-iodomélatonine comme radioligand de référence. La radioactivité retenue est déterminée à l'aide d'un compteur à scintillation liquide.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

### 2. Etude de liaison aux sites de liaisons MT₃ de la melatonine

Les expériences de liaison sur les sites *MT*_{*3*} sont réalisées sur membranes de cerveau de hamster en utilisant la 2-[¹²⁵I] iodomélatonine comme radioligand. Les membranes sont incubées pendant 30 minutes avec 1a 2-[¹²⁵I] iodomélatonine à la température de 4°C et différentes concentrations des composés à tester. Après l'incubation, les membranes sont rapidement filtrées puis lavées par du tampon froid à l'aide d'un système de filtration. La radioactivité fixée est mesurée par un compteur à scintillation. Les valeurs d'IC₅₀ (concentration inhibant de 50 % la liaison spécifique) sont calculées à partir des courbes de compétition selon un modèle de régression non linéaire.

Ainsi, les valeurs d'IC₅₀ trouvées pour les composés de l'invention attestent d'une liaison pour l'un ou l'autre des sous-types réceptoriels, ces valeurs étant ≤ 10 µM.

### EXEMPLE D: Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention pennettent d'agir de façon puissante sur le rythme circadien *via* le système mélatoninergique.

### EXEMPLE E : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE F : Activité des composés de l'invention sur l'artère caudale de rat

Les composés de l'invention ont été testés *in vitro* sur l'artère caudale de rat. Les récepteurs mélatoninergiques sont présents sur ces vaisseaux ce qui en fait un modèle pharmacologique relevant pour étudier l'activité de ligands mélatoninergiques. La stimulation des récepteurs peut induire soit une vasoconstriction soit une dilatation en fonction du segment artériel étudié.

### Protocole

Des rats âgés de 1 mois sont habitués pendant 2 à 3 semaines à un cycle lumière/obscurité 12h/12h.
Après sacrifice, l'artère caudale est isolée et maintenue dans un milieu richement oxygéné. Les artères sont ensuite cannulées aux deux extrémités, suspendues verticalement dans une chambre d'organe dans un milieu approprié et perfusées via leur extrémité proximale. Les changements de pression dans le débit de la perfusion permettent d'évaluer l'effet vasoconstricteur ou vasodilatateur des composés.
L'activité des composés est évaluée sur des segments pré-contractés par la phényléphrine (1 µM). Une courbe concentration-réponse est déterminée de façon non-cumulative par addition d'une concentration du composé étudié sur le segment pré-contracté. Lorsque l'effet observé a atteint l'équilibre, le milieu est changé et la préparation laissée 20 minutes avant l'addition d'une même concentration de phényléphrine et d'une nouvelle concentration du composé étudié.

### Résultats

Les composés de l'invention modifient de façon significative le diamètre des artères caudales préconstrictées par la phényléphrine.

### EXEMPLE G : Composition pharmaceutique : Comprimés

| | |
|---|---|
| 1000 comprimés dosés à 5 mg de 3-{2-[(Cyclopropylcarbonyl)amino]éthyl}-1-benzofuran-5-yl-carbamate de méthyle (Exemple 75) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I):
R-A-R' (I)
dans laquelle :
◆ A représente :
- un système cyclique de formule (II) : 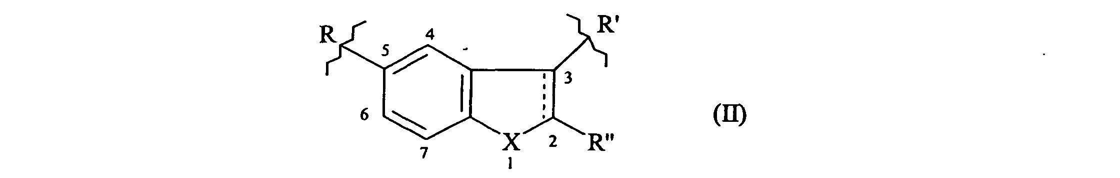 où
• X représente un atome d'oxygène ou de soufre, ou un groupement NR₀ (dans lequel R₀ représente un atome d'hydrogène ou un groupement alkyle (C₁-C₆) linéaire ou ramifié, aryle, arylalkyle (C₁-C₆) linéaire ou ramifié ou SO₂Ph),
• la représentation ---- signifie que la liaison peut être simple ou double étant entendu que la valence des atomes est respectée,
• R" représente un groupement Rₐ, ORₐ, CORₐ, COORₐ, OCORₐ, OSO₂CF₃, ou atomes d'halogène,
où Rₐ représente un atome d'hydrogène, un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, polyhalogénoalkyle (C₁-C₆) linéaire ou ramifié, cycloalkyle (C₃-C₈) substitué ou non, cycloalkyle (C₃-C₈) alkyle (C₁-C₆) linéaire ou ramifié substitué ou non, cycloalkényle (C₃-C₈) substitué ou non, cycloalkényle (C₃-C₈) alkyle (C₁-C₆) linéaire ou ramifié substitué ou non, aryle, arylalkyle (C₁-C₆) linéaire ou ramifié, arylalkényle (C₁-C₆) linéaire ou ramifié, hétéroaryle, hétéroarylalkyle (C₁-C₆) linéaire ou ramifié, hétéroarylalkényle (C₁-C₆) linéaire ou ramifié, hétérocycloalkyle substitué ou non, hétérocycloalkényle substitué ou non, hétérocycloalkylalkyle (C₁-C₆) linéaire ou ramifié substitué ou non, ou hétérocycloalkényl alkyle (C₁-C₆) linéaire ou ramifié substitué ou non,
- ou un système cyclique de formule (III) : 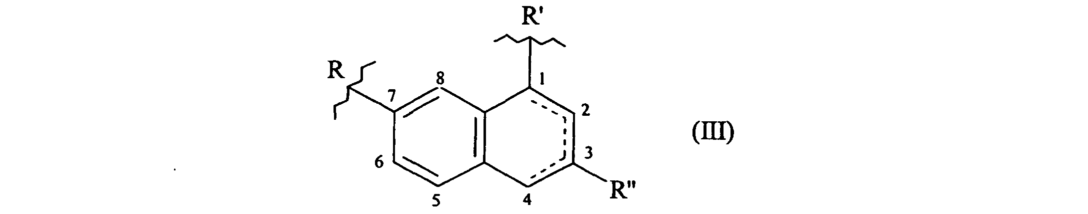 où R" et la représentation ----- ont la même signification que précédemment,
◆ R représente :
un groupement de formule (VI) : 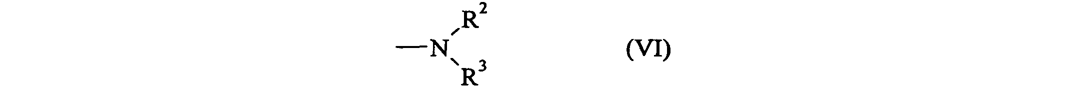 où
• R² représente un groupement Rₐ tel que défini précédemment,
• R³ représente un groupement COR'ₐ, CSR'ₐ, CONR'ₐR"ₐ, CSNR'ₐR"ₐ, COOR'ₐ, CSOR'ₐ ou S(O)ᵥR'ₐ (dans lesquels R'ₐ et R"ₐ, identiques ou différents, peuvent prendre toutes les valeurs de Rₐ défini précédemment et peuvent également former, avec l'atome d'azote qui les porte un groupement cyclique contenant 5 à 10 chaînons et pouvant comporter, en plus de l'atome d'azote par lequel il est relié, un à trois hétéroatomes choisis parmi oxygène, soufre et azote, et v vaut 1 ou 2),
◆ et R' représente un groupement de formule (IX) :
―G―R⁵ (IX)
où
• G représente une chaîne alkylène -(CH₂)ₜ- (dans laquelle t vaut 2 ou 3),
• et R⁵ représente un groupement 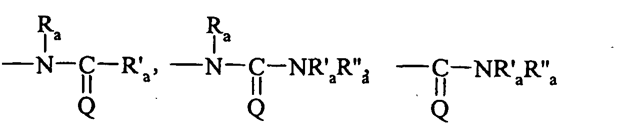 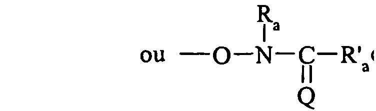 dans lesquels Q représente un atome d'oxygène ou de soufre, et Rₐ, R'ₐ et R"ₐ (identiques ou différents) sont définis de la même façon que précédemment, R'ₐ et R"ₐ pouvant former avec l'atome d'azote qui les porte un groupement cyclique tel que défini précédemment,
étant entendu que :
- par "hétérocycloalkyle" on entend tout groupement saturé mono ou polycyclique contenant de 5 à 10 atomes contenant 1 à 3 hétéroatomes choisis parmi azote, oxygène ou soufre,
- par "hétérocycloalkényle" on entend tout groupement mono ou polycyclique non aromatique contenant une ou plusieurs insaturations, contenant de 5 à 10 atomes et pouvant contenir 1 à 3 hétéroatomes choisis parmi azote, oxygène ou soufre,
- le terme "substitué" affecté aux expressions "alkyle", "alkényle", "alkynyle", signifie que ces groupements sont substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
- le terme "substitué" affecté aux expressions "cycloalkyle", "cycloalkylalkyle", "cycloalkényle", "cycloalkénylalkyle", "hétérocycloalkyle", "hétérocycloalkényle", "hétérocycloalkylalkyle", "hétérocycloalkénylalkyle", signifie que la partie cyclique de ces groupements est substituée par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
- par "aryle" on entend tout groupement aromatique mono ou polycyclique contenant 6 à 22 atomes de carbone, ainsi que le groupement biphényle,
- par "hétéroaryle" on entend tout groupement aromatique mono ou polycyclique contenant de 5 à 10 atomes contenant 1 à 3 hétéroatomes choisis parmi azote, oxygène ou soufre,
les groupements "aryle" et "hétéroaryle" pouvant être substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, cyano, carboxy, nitro, amino ou atomes d'halogène,
étant entendu que :
- lorsque A représente un noyau indolique, R" représente un atome d'hydrogène,
- lorsque A représente un noyau indolique et R représente un groupement -NHCOR'ₐ,
- NHCOOR'ₐ ou NHCONR'ₐR"ₐ, alors G-R⁵ ne peut représenter un groupement -(CH₂)₂-NHCOR_{b} dans lequel R_{b} représente un groupement alkyl (C₁-C₄) ou CF₃,
- lorsque A représente un noyau benzofurane ou benzothiophène, R" est différent d'un groupement COPh (où Ph est substitué ou non),
- lorsque A représente un noyau benzofurane ou benzothiophène, R ne peut représenter un groupement -NRₐCOR_{c}, -NHSO₂R_{c}, -NHCOCH₂R_{c}, ou NHCONHR_{c} où R_{c} représente un groupement hétérocyclique ou aryle,
- lorsque A représente un tétrahydronaphtalène, R⁵ ne peut représenter un groupement CONR'ₐR"ₐ tel que défini précédemment,
leurs énantiomères et diastéréoisomères, ainsi que leurs sels d'addition à un acide ou une base pharmaceutiquement acceptable.

2. Composés de formule (I) selon la revendication 1 tels que R représente un groupement de formule (VI) dans lequel R³ représente un groupement COR'ₐ où R'ₐ est tel que défini dans la revendication 1, leurs énantiomères et diastéréoisomères, ainsi que leurs sels d'addition à un acide ou une base pharmaceutiquement acceptable.

3. Composés de formule (I) selon la revendication 1 tels que R représente un groupement de formule (VI) dans lequel R³ représente un groupement COOR'ₐ où R'ₐ est tel que défini dans la revendication 1, leurs énantiomères et diastéréoisomères, ainsi que leurs sels d'addition à un acide ou une base pharmaceutiquement acceptable.

4. Composés de formule (I) selon la revendication 1 tels que R' représente un groupement G-R⁵ dans lequel G représente une chaîne alkylène -(CH₂)ₜ- où t vaut 2 ou 3 et R⁵ représente un groupement 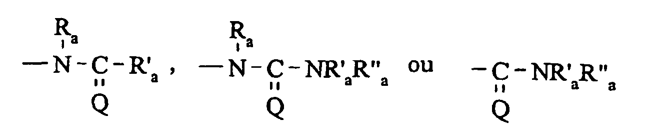 dans lesquels Rₐ, R'ₐ, R"ₐ et Q sont tels que définis dans la revendication 1, leurs énantiomères et diastéréoisomères, ainsi que leurs sels d'addition à un acide ou une base pharmaceutiquement acceptable.

5. Composés de formule (I) selon la revendication 1 tels que R' représente un groupement G-R⁵ dans lequel R⁵ représente un groupement -NHCOR'ₐ ou -CONHR'ₐ où R'ₐ est tel que défini dans la revendication 1, leurs énantiomères et diastéréoisomères, ainsi que leurs sels d'addition à un acide ou une base pharmaceutiquement acceptable.

6. Composés de formule (I) selon la revendication 1 tels que A représente un système cyclique de formule (II), leurs énantiomères et diastéréoisomères, ainsi que leurs sels d'addition à un acide ou une base pharmaceutiquement acceptable.

7. Composés de formule (I) selon la revendication 1 tels que A représente un système cyclique de formule (III), leurs énantiomères et diastéréoisomères, ainsi que leurs sels d'addition à un acide ou une base pharmaceutiquement acceptable.

8. Composés de formule (I) selon la revendication 1 tels que A représente un système cyclique de formule (II), R représente groupement -NHCOR'ₐ a où R'ₐ a est tel que défini dans la revendication 1, et R' représente un groupement de formule (IX) où R⁵ représente un groupement 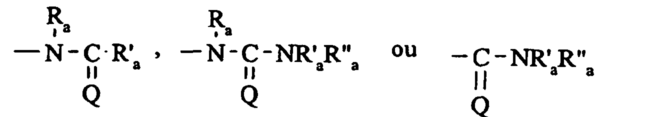 où Q, Rₐ, R'ₐ et R"ₐ sont tels que définis dans la revendication 1, leurs énantiomères et diastéréoisomères, ainsi que leurs sels d'addition à un acide ou une base pharmaceutiquement acceptable.

9. Composés de formule (I) selon la revendication 1 tels que A représente un système cyclique de formule (II), R représente un groupement -NHCOOR'ₐ où R'ₐ est tel que défini dans la revendication 1, et R' représente, un groupement de formule (IX) où R⁵ représente un groupement 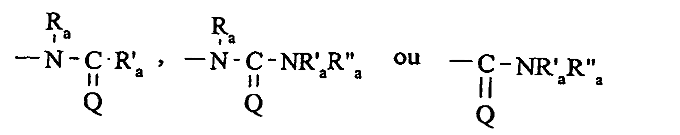 où Q, Rₐ, R'ₐ et R"ₐ sont tels que définis dans la revendication 1, leurs énantiomères et diastéréoisomères, ainsi que leurs sels d'addition à un acide ou une base pharmaceutiquement acceptable.

10. Composés de formule (I) selon la revendication 1 tels que A représente un système cyclique de formule (III), R représente un groupement -NHCOR'ₐ où R'ₐ est tel que défini dans la revendication 1, et R' représente un groupement de formule (IX) où R⁵ représente un groupement 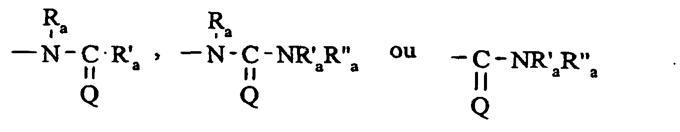 où Q, Rₐ, R'ₐ et R"ₐ sont tels que définis dans la revendication 1, leurs énantiomères et diastéréoisomères, ainsi que leurs sels d'addition à un acide ou une base pharmaceutiquement acceptable.

11. Composés de formule (I) selon la revendication 1 tels que A représente un système cyclique de formule (III), R représente un groupement -NHCOOR'ₐ où R'ₐ est tel que défini dans la revendication 1, et R' représente un groupement de formule (IX) où R⁵ représente un groupement 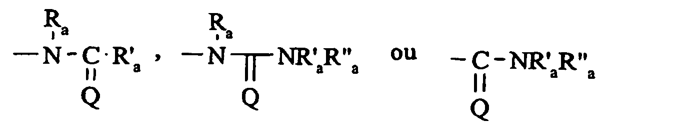 où Q, Rₐ, R'ₐ et R"ₐ sont tels que définis dans la revendication 1, leurs énantiomères et diastéréoisomères, ainsi que leurs sels d'addition à un acide ou une base pharmaceutiquement acceptable.

12. Composés de formule (I) selon la revendication 1 tels que A représente un noyau naphtalène, dihydro ou tétrahydronaphtalène, leurs énantiomères et diastéréoisomères, ainsi que leurs sels d'addition à un acide ou une base pharmaceutiquement acceptable.

13. Composés de formule (I) selon la revendication 1 tels que A représente un noyau benzofurane ou dihydrobenzofurane, leurs énantiomères et diastéréoisomères, ainsi que leurs sels d'addition à un acide ou une base pharmaceutiquement acceptable.

14. Composés de formule (I) selon la revendication 1 tels que A représente un noyau benzothiophène ou dihydrobenzothiophène, leurs énantiomères et diastéréoisomères, ainsi que leurs sels d'addition à un acide ou une base pharmaceutiquement acceptable.

15. Composés de formule (I) selon la revendication 1 tels que A représente un noyau indole ou indoline, leurs énantiomères et diastéréoisomères, ainsi que leurs sels d'addition à un acide ou une base pharmaceutiquement acceptable.

16. Composés de formule (I) selon la revendication 1 tels que A représente un noyau naphtalène, dihydro ou tétrahydronaphtalène, R représente un groupement -NHCORₐ, où Rₐ est tel que défini dans la revendication 1, et R' représente un groupement -(CH₂)ₜ-NHCOR'ₐ ou -(CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

17. Composés de formule (I) selon la revendication 1 tels que A représente un noyau benzofurane ou dihydrobenzofurane, R représente un groupement -NHCORₐ, où Rₐ est tel que défini dans la revendication 1, et R' représente un groupement -(CH₂)ₜ-NHCOR'ₐ ou - (CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

18. Composés de formule (I) selon la revendication 1 tels que A représente un noyau benzothiophène ou dihydrobenzothiophène, R représente un groupement -NHCORₐ, où Rₐ est tel que défini dans la revendication 1, et R' représente un groupement -(CH₂)ₜNHCOR'ₐ ou -(CH₂)ₜCONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

19. Composés de formule (I) selon la revendication 1 tels que A représente un noyau indole ou indoline, R représente un groupement -NHCORₐ, où Rₐ est tel que défini dans la revendication 1, et R' représente un groupement -(CH₂)ₜ-NHCOR'ₐ ou -(CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

20. Composé de formule (I) selon la revendication 1 tel que A représente un noyau naphtalène, dihydronaphtalène ou tétrahydronaphtalène, R représente un groupement -NHCOORₐ ou -N(alk)COORₐ où Rₐ est tel que défini dans la revendication 1 et alk représente un groupement alkyle, et R' représente un groupement -(CH₂)ₜ-NHCOR'ₐ ou -(CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

21. Composés de formule (I) selon la revendication 1 tels que A représente un noyau benzofurane ou dihydrobenzofurane, R représente -NHCOORₐ ou -N(alk)COORₐ où Rₐ est tel que défini dans la revendication 1 et alk représente un groupement alkyle, et R' représente un groupement -(CH₂)ₜ-NHCOR'ₐ ou -(CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

22. Composés de formule (I) selon la revendication 1 tels que A représente un noyau benzothiophène ou dihydrobenzothiophène, R représente un groupement -NHCOORₐ ou -N(alk)COORₐ où Rₐ est tel que défini dans la revendication 1 et alk représente un groupement alkyle, et R' représente un groupement -(CH₂)ₜ-NHCOR'ₐ ou -(CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

23. Composés de formule (I) selon la revendication 1 tels que A représente un noyau indole ou indoline, R représente un groupement -NHCOORₐ ou -N(alk)COORₐ où Rₐ est tel que défini dans la revendication 1 et alk représente un groupement alkyle, et R' représente un groupement -(CH₂)ₜ-NHCOR'ₐ ou -(CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

24. Composés de formule (I) selon la revendication 1 qui sont :
* le N-{3-[2-(Acétylamino)éthyl]-1-benzofuran-5-yl}-2,2,2-trifluoroacétamide,
* le N-{2-[5-(Acétylamino)-1-benzofuran-3-yl]éthyl}cyclopropane carboxamide,
* le N-{2-[5-(Acétylamino)-1-benzothiophèn-3-yl]éthyl}benzamide,
leurs énantiomères et diastéréoisomères, ainsi que leurs sels d'addition à un acide ou une base pharmaceutiquement acceptable.

25. Composés de formule (I) selon la revendication 1 qui sont :
* le N-{8-[2-([2-Phénylacétyl]amino)éthyl]-2-naphtyl}butanamide,
* le N-(8-{2-[(2-Bromoacétyl)amino]éthyl}-2-naphtyl)-1-cyclohexane carboxamide,
* le N-{8-[2-(Heptanoylamino)éthyl]-2,6-dinaphtyl}-2-butènamide,
* le N-{8-[2-(Acétylamino)éthyl]-2-naphtyl}acétamide,
leurs énantiomères et diastéréoisomères, ainsi que leurs sels d'addition à un acide ou une base pharmaceutiquement acceptable.

26. Composés de formule (I) selon la revendication 1 qui sont :
* le N-{3-[2-(Acétylamino)éthyl]benzo[*b*]furan-5-yl}carbamate de méthyle,
* le 3-{2-[(Cyclopropylcarbonyl)amino]éthyl}-1-benzofuran-5-yl-carbamate de méthyle,
* le 3-[2-(Acétylamino)éthyl]-1-benzofuran-5-yl-carbamate de tert-butyle,
* le 3-[2-(Acétylamino)éthyl]-1-benzofuran-5-yl-(méthyl)carbamate de tert-butyle,
* le 3-[2-(Benzoylamino)éthyl]-1-benzofuran-5-yl carbamate de méthyle,
* le 3-[2-(Isobutyrylamino)éthyl]-1-benzofuran-5-yl carbamate de méthyle,
* le 3-[2-(2-Furoylamino)éthyl]-1-benzofuran-5-yl carbamate de méthyle,
* le 3-{2-[(Cyclopentylcarbonyl)amino]éthyl}-1-benzofuran-5-yl carbamate de méthyle,
leurs énantiomères et diastéréoisomères, ainsi que leurs sels d'addition à un acide ou une base pharmaceutiquement acceptable.

27. Composés de formule (I) selon la revendication 1 qui sont :
* le N-(8-{2-[(2-Bromoacétyl)amino]éthyl}-2-naphtyl)carbamate d'éthyle,
* le N-{8-[2-(Acétylamino)éthyl]-6-phényl-2-naphtyl}carbamate de méthyle,
* le N-{8-[2-(Acétylamino)éthyl]-5,6,7,8-tétrahydro-2-naphtalényl}carbamate d'hexyle,
* le 8-[2-(Acétylamino)éthyl]-2-naphtyl carbamate de méthyle
leurs énantiomères et diastéréoisomères, ainsi que leurs sels d'addition à un acide ou une base pharmaceutiquement acceptable.

28. Procédé de préparation des composés de formule (I) **caractérisé en ce que** l'on utilise comme produit de départ le composé de formule (X) : 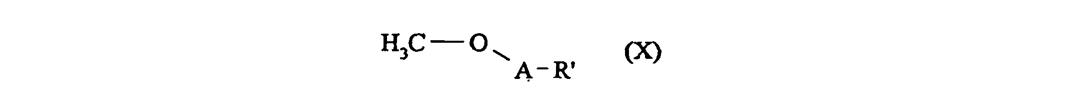 dans laquelle A et R' sont tels que définis dans la revendication 1, que l'on soumet à une déméthylation en utilisant des agents classiques comme HBr, AlCl₃, AlBr₃, BBr₃ ou des systèmes binaires acides de Lewis/nucléophiles comme AlCl₃/PhCH₂SH, ou BBr₃/Me₂S par exemple, pour obtenir le composé de formule (XI) :
HO ― A - R' (XI)
dans laquelle A et R' sont définis comme précédemment,
que l'on transforme, grâce à l'action de réactifs tels que POCl₃, PCl₅, Ph₃PBr₂, PhPCl₄, HBr ou HI en dérivé halogéné correspondant de formule (XII) :
Hal-A-R' (XII)
dans laquelle A et R' sont définis comme précédemment et Hal représente un atome d'halogène (les composés de formule (XII) pouvant être obtenus par des réactions d'échange comme par exemple le traitement d'un dérivé chloré par KF au sein de la diméthylformamide pour conduire au dérivé fluoré correspondant, ou le traitement d'un dérivé bromé par KI en présence de sels de cuivre pour conduire au dérivé iodé correspondant),
qui est traité :
par du monoxyde de carbone et Bu₃SnH, la réaction étant catalysée par du palladium (0), pour conduire à l'aldéhyde correspondant de formule (XIII) : 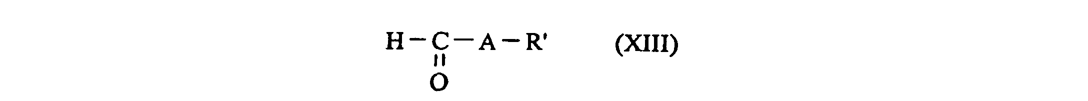 dans laquelle A et R' sont tels que définis précédemment,
composé de formule (XIII) pouvant par ailleurs être obtenu par des techniques classiques de lithiations à partir du dérivé halogéné de formule (XII), ou par l'intermédiaire du dérivé vinylique correspondant (obtenu à partir du composé de formule (XII) par action de vinyltributylétain et de palladium tetrakis) soumis à une ozonolyse, ou encore par formylation directe du noyau A selon une réaction de Vilsmeier par exemple,
composé de formule (XIII) que l'on soumet à un agent oxydant pour obtenir le composé de formule (XIV) :
HOOC―A-R' (XIV)
dans laquelle A et R' sont définis comme précédemment, qui est :
transformé, après l'action de chlorure de thionyle et d'un azidure, puis d'un acide, en composé de formule (XV) :
H₂N―A-R' (XV)
dans laquelle A et R' sont définis comme précédemment, sur lequel on condense :
- soit un chlorure d'acyle CICORₐ ou l'anhydride (mixte ou symétrique) correspondant pour lesquels Rₐ est tel que défini précédemment, pour conduire au composé de formule (I/c), cas particulier des composés de formule (I) : 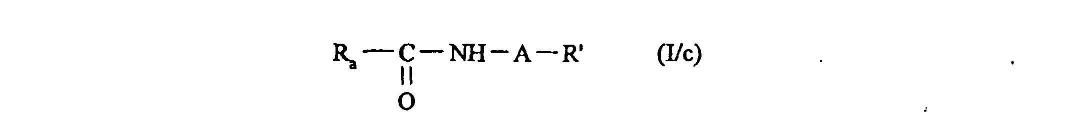 dans laquelle Rₐ, A et R' sont définis comme précédemment,
qui peut être soumis à l'action d'un composé de formule (XVI) :
R¹ ₐ―J (XVI)
dans laquelle R¹ₐ peut prendre toutes les valeurs de Rₐ à l'exception de l'atome d'hydrogène et J représente un groupe partant comme un atome d'halogène ou un groupement tosyle,
pour obtenir le composé de formule (I/d), cas particulier des composés de formule (I) : 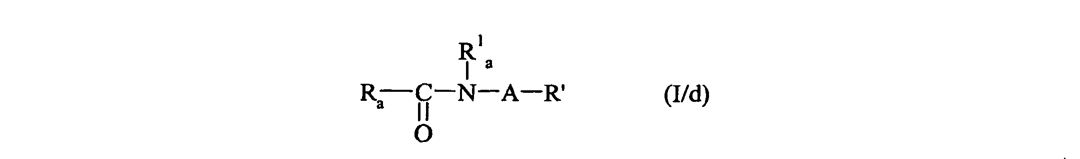 dans laquelle Rₐ, R¹ₐ, A et R' sont tels que définis précédemment,
les composés de formule (I/c) et (I/d) formant le composé de formule (I/e), cas particulier des composés de formule (I) : 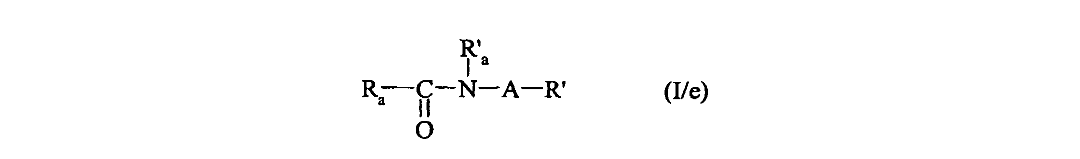 dans laquelle Rₐ, R'ₐ, A et R' ont la même définition que précédemment,
composé de formule (I/e) que l'on peut soumettre à un agent de thionation tel que le réactif de Lawesson par exemple afin d'obtenir le composé de formule (I/f), cas particulier des composés de formule (I) : 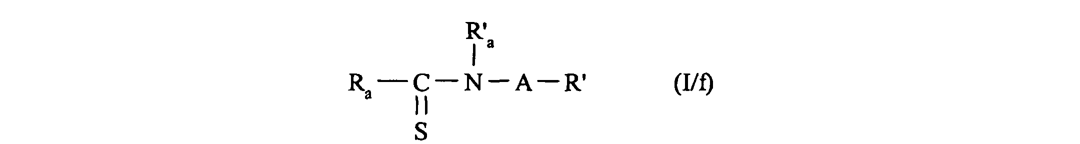 dans laquelle Rₐ, R'ₐ, A et R' sont définis comme précédemment,
- soit un composé de formule (XVII) :
Q=C=N-R'ₐ (XVII)
dans laquelle Q et R'ₐ sont tels que définis précédemment,
pour conduire au composé de formule (I/g), cas particulier des composés de formule (I) : 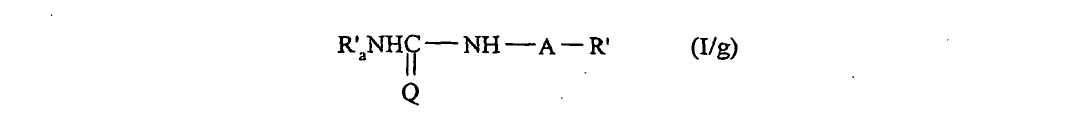 dans laquelle R'ₐ, Q, A et R' sont définis comme précédemment,
qui peut être soumis à l'action d'un composé de formule (XVI) pour obtenir le composé de formule (I/h), cas particulier des composés de formule (I) : 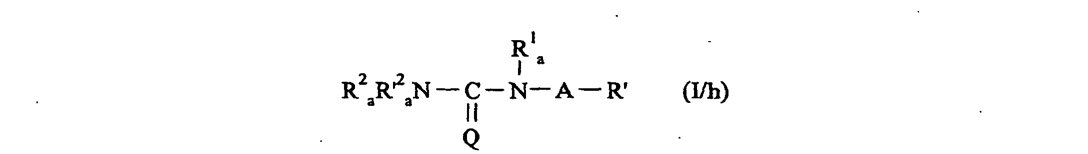 dans laquelle Q, R¹ₐ, A et R' ont la même définition que précédemment et R²ₐ et R^{,2}ₐ, identiques
ou différents, peuvent prendre toutes les valeurs de Rₐ à l'exception de l'atome d'hydrogène et ne peuvent pas former de structure cyclique avec l'atome d'azote qui les porte,
- soit un composé de formule (XVIII): 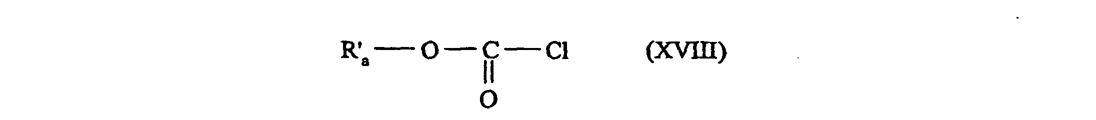 dans laquelle R'ₐ est tel que défini précédemment, ou son anhydride correspondant (R'ₐOCO)₂O
pour obtenir le composé de formule (I/i), cas particulier des composés de formule (I) : 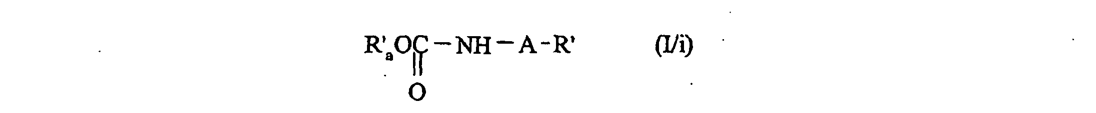 dans laquelle R'ₐ, A et R' sont définis comme précédemment;
qui peut être soumis à l'action d'un composé de formule (XVI) et/ou à l'action d'un agent de thionation afin de conduire au composé de formule (I/j), cas particulier des composés de formule (I) : 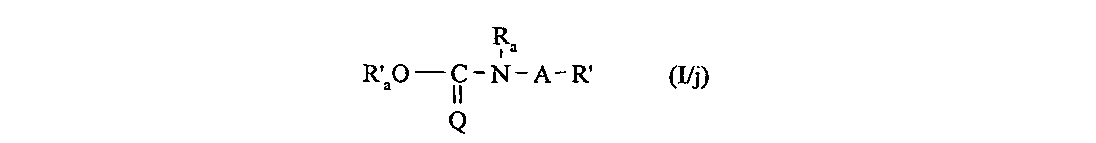 dans laquelle Rₐ, R'ₐ, Q, A et R' sont définis comme précédemment,
- soit un composé de formule (XIX) :
RₐSO₂Cl (XIX)
dans laquelle Rₐ est tel que défini précédemment,
suivi éventuellement de l'action d'un composé de formule (XVI) pour conduire au composé de formule (I/k), cas particulier des composés de formule (I) : 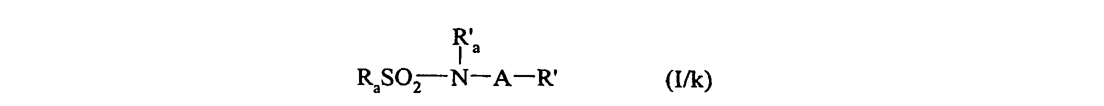 dans laquelle Rₐ, R'ₐ, A et R' sont définis comme précédemment,
les composés (I/c) à (I/k) pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

29. Procédé de préparation des composés de formule (I) selon la revendication 1 **caractérisé en ce que** l'on utilise comme produit de départ le composé de formule (XXII) : 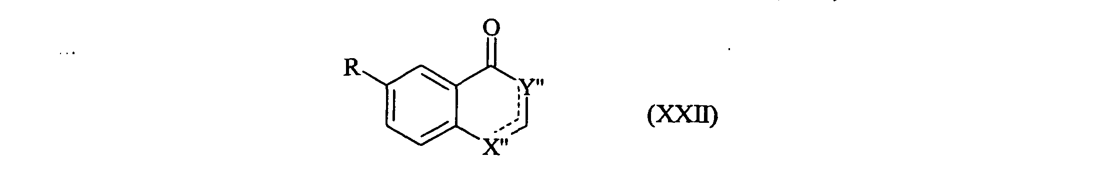 dans laquelle R et la représentation .... sont définis comme dans la revendication 1, Y" représente une liaison et dans ce cas X" représente un atome d'oxygène, ou de soufre ou un groupement NR₀ (où R₀ est tel que défini précédemment), ou X" et Y", identiques ou différents, représentent un groupement C(H)q (ou q vaut 1 ou 2),
que l'on soumet à une réaction de Wittig puis à une réduction afin de conduire au composé de formule (XXIII) : 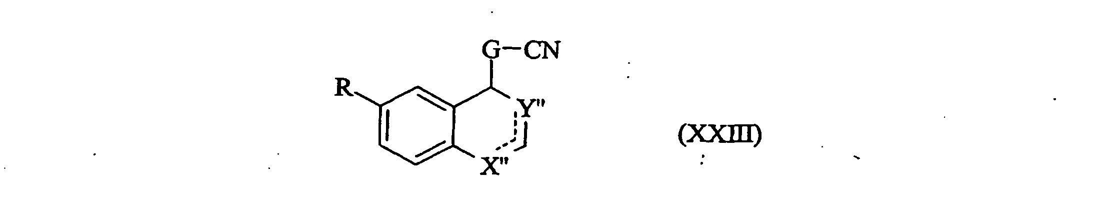 dans laquelle R, X", Y", G et la représentation .... sont définis comme précédemment,
qui peut être oxydé afin de conduire au composé de formule (XXIV) : 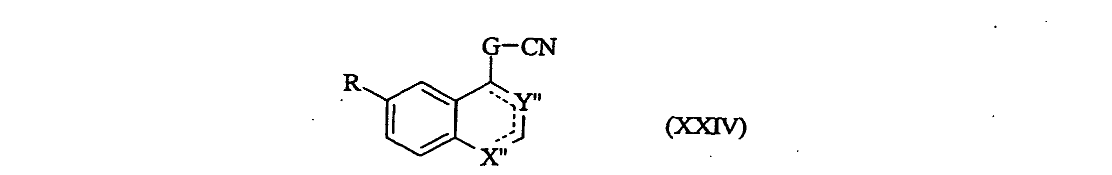 dans laquelle R, X", Y", G et la représentation .... ont la même définition que précédemment, qui est
* soit hydrolysé en milieu acide ou basique puis soumis, après activation sous forme de chlorure d'acide ou en présence d'un agent de couplage, à l'action d'une amine HNR'ₐR"ₐ dans laquelle R'ₐ et R"ₐ sont définis comme précédemment afin de conduire au composé de formule (I/p), cas particulier des composés de formule (I) : 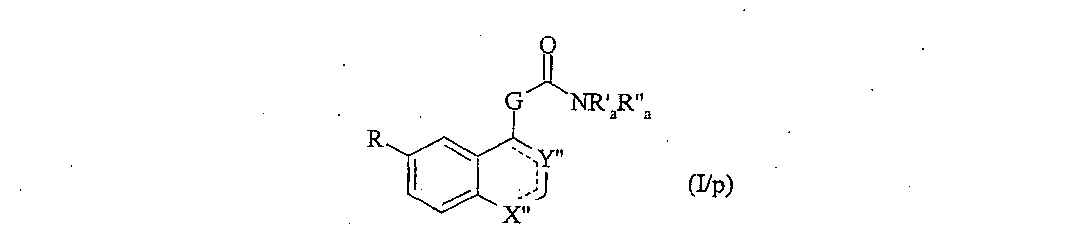 dans laquelle R, X", Y", G, R'ₐ, R"ₐ et la représentation .... sont définis comme précédemment,
que l'on peut soumettre à un agent de thionation comme le réactif de Lawesson pour conduire au composé de formule (I/q), cas particulier des composés de formule (I) : 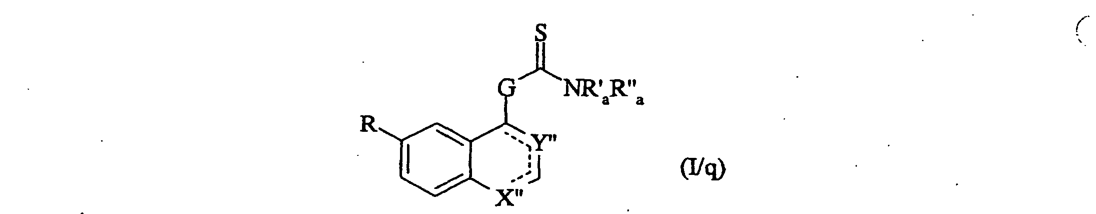 dans laquelle R, X", Y", G, R'ₐ, R"ₐ et la représentation .... sont définis comme précédemment,
* soit hydrolysé en milieu acide ou basique puis transformé en azide correspondant pour conduire, après avoir subi un réarrangement de Curtius et une hydrolyse, au composé de formule (XXV) : 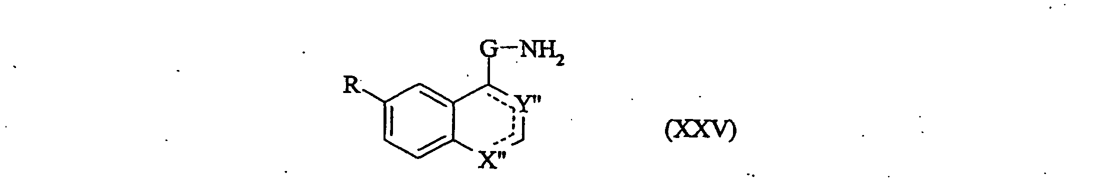 dans laquelle R, X", Y" et G sont tels que définis précédemment,
qui est mis en réaction avec :
- un chlorure d'acyle ClCOR'ₐ ou l'anhydride (mixte ou symétrique) correspondant pour lesquels R'ₐ est tel que défini précédemment, suivi éventuellement de l'action d'un composé de formule (XVI) et/ou de l'action d'un agent de thionation afin de conduire au composé de formule (I/r), cas particulier des composés de formule (I) : 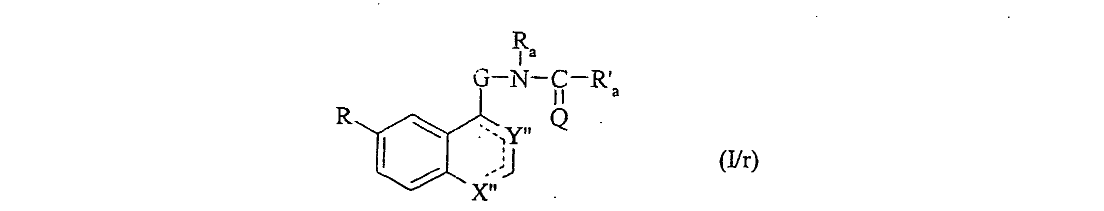 dans laquelle R, X", Y", G, Rₐ, R'ₐ, Q et la représentation .... sont définis comme précédemment,
- ou avec un composé de formule (XVII) suivi éventuellement de l'action d'un composé de formule (XVI) afin de conduire au composé de formule (I/s), cas particulier des composés de formule (I) : 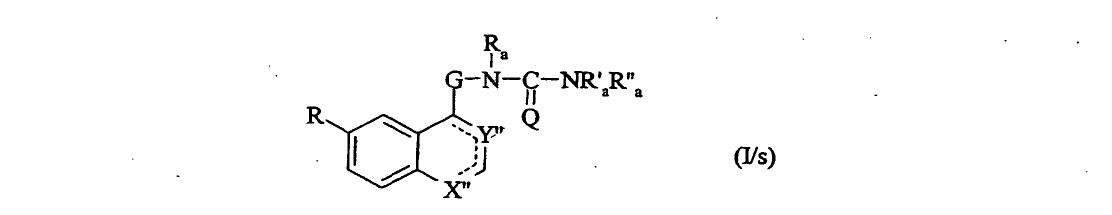 dans laquelle R, X", Y", G, Rₐ, R'ₐ, R"ₐ, Q et la représentation .... sont définis comme précédemment,
les composés (I/p) à (I/s) pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

30. Compositions pharmaceutiques contenant les produits de formule (I) selon l'une quelconque des revendications 1 à 27 ou un de leurs sels d'addition avec un acide ou une base pharmaceutiquement acceptable en combinaison avec un ou plusieurs excipients pharmaceutiquement acceptables.

31. Compositions pharmaceutiques selon la revendication 30 utiles à la fabrication de médicaments pour le traitement des troubles liés au système mélatoninergique.

## Patentansprüche

1. Verbindung der. Formel (I):
R- A - R' (I)
in der:
◆ A:
- ein cyclisches System der Formel (II): 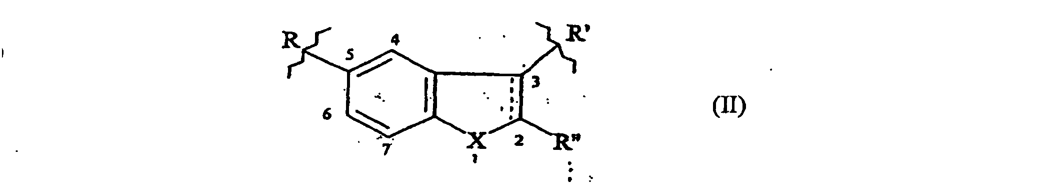 in der
• X ein Sauerstoff- oder Schwefelatom oder eine Gruppe NRₒ darstellt (worin Rₒ ein Wasserstoffatom oder eine geradkettige oder verzweigte (C₁-C₆)-Alkylgruppe. Arylgruppe, geradkettige oder verzweigte Aryl-(C₁-C₆)-alkylgruppe oder Gruppe SO₂Ph darstellt).
• das Symbol ---- bedeutet, daß -die Bindung einfach oder doppelt sein kann, mit der Maßgabe, daß die Wertigkeit der Atome berücksichtigt ist,
• R" eine Gruppe Rₐ, ORₐ, CORₐ, COORₐ, OCORₐ, OSO₂CF₃, oder Halogenatome bedeutet,
worin Rₐ ein Wasserstoffatom, eine geradkettige oder verzweigte, substituierte oder nichtsubstituierte (C₁-C₆)-Alkylgruppe, geradkettige oder verzweigte, substituierte oder nichtsubstituierte (C₂-C₆)-Alkenylgruppe, geradkettige oder verzweigte, substituierte oder nichtsubstituierte (C₂-C₆)-Alkinylgruppe, geradkettige oder verzweigte (C₁-C₆)-Polyhalogenalkylgruppe, substituierte oder nichtsubstituierte (C₃-C₈)-Cycloalkylgruppe, geradkettige oder verzweigte, substituierte oder nichtsubstituierte (C₃-C₈)-Cycloalkyl-(C₁-C₆)-alkylgruppe, substituierte oder nichtsubstituierte (C₃-C₈)-Cycloalkenylgruppe, geradkettige oder verzweigte, substituierte oder unsubstituierte (C₃-C₈) - Cycloalkenyl - (C₁-C₆)-alkylgruppe , Arylgruppe , geradkettige oder verzweigte Aryl - (C₁-C₆) - alkylgruppe, geradkettige oder verzweigte Aryl - (C₁-C₆) - alkenylgruppe, Heteroarylgruppe, geradkettige oder verzweigte Heteroaryl - (C₁-C₆) - alkylgruppe , geradkettige oder verzweigte Heteroaryl - (C₁-C₆) - alkenylgruppe , substituierte oder unsubstituierte Heterocycloalkylgruppe , substituierte oder unsubstituierte Heterocycloalkenylgruppe, geradkettige oder verzweigte, substituierte oder unsubstituierte Heterocycloalkyl-(C₁-C₆)-alkylgruppe oder geradkettige oder verzweigte, substituierte oder unsubstituierte Heterocycloalkenyl-(C₁-C₆)-alkylgruppe darstellt,
- oder ein cyclisches System der Formel (III) bedeutet: 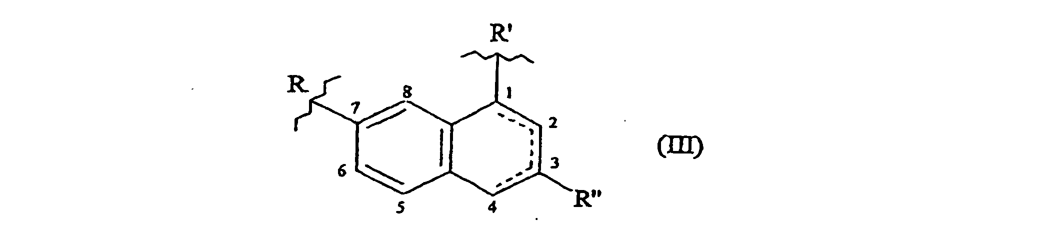 worin R" und das Symbol ----- die oben angegebenen Bedeutungen besitzen,
◆ R:
eine Gruppe der Formel (VI) bedeutet: 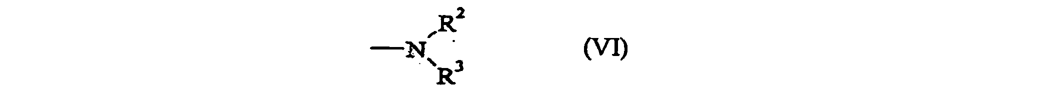 in der
• R² eine Gruppe Rₐ darstellt, wie sie oben definiert ist,
• R³ eine Gruppe COR'ₐ, CSR'ₐ, CONR'ₐR"ₐ, CSNR'ₐR"ₐ, COOR'ₐ, CSOR'ₐ oder S(O)ᵥR'ₐ (worin R'ₐ und R"ₐ, die gleichartig oder verschieden sind, die Rₐ oben angegebenen Bedeutungen besitzen und weiterhin zusammen mit dem sie tragenden Stickstoffatom eine cyclische Gruppe bilden können, die 5 bis 10 Kettenglieder aufweisen kann und zusätzlich zu dem Stickstoffatom ein bis drei Heteroatome ausgewählt aus Sauerstoff, Schwefel und Stickstoff enthalten kann, und v den Wert 1 oder 2 besitzt),
◆ und R' eine gruppe der Formel (IX) bedeutet :
- G - R⁵ (IX)
worin
• G eine Alkylenkette -(CH₂)ₜ- darstellt (worin t den Wert 2 oder 3 besitzt),
• und R⁵ eine Gruppe 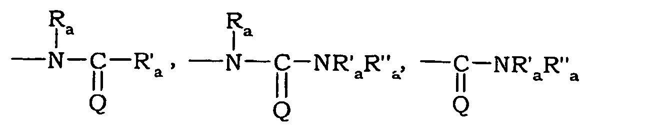 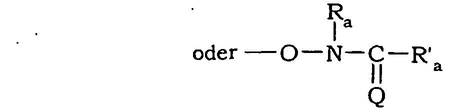 darstellt, worin Q ein Sauerstoff- oder Schwefelatom darstellt, und Rₐ, R'ₐ und R"ₐ (die gleichartig oder verschieden sind) die oben angegebenen Bedeutungen besitzen und R'ₐ und R"ₐ zusammen mit dem sie tragenden Stickstoffatom eine cyclische Gruppe bilden können, wie sie oben definiert worden ist, mit der Maßgabe, daß:
- man unter "Heterocycloalkyl" jede gesättigte, mono- oder polycyclische Gruppe, die 5 bis 10 Atome enthält, die 1 bis 3 Heteroatome ausgewählt aus Stickstoff, Sauerstoff oder Schwefel umfassen, versteht,
- man unter "Heterocycloalkenyl" jede mono- oder polycyclische nichtaromatische Gruppe, die eine oder mehrere Unsättigungen aufweist und 5 bis 10 Atome enthält, die 1 bis 3 Heteroatome ausgewählt aus Stickstoff. Sauerstoff oder Schwefel enthalten können, versteht,
- der Begriff "substituiert" unter Bezugnahme auf die Begriffe "Alkyl", "Alkenyl", "Alkinyl" bedeutet, daß diese Gruppen durch einen oder mehrere gleiche oder verschiedenartige Reste ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Amino oder Halogenatomen substituiert sind,
- der Begriff "substituiert" unter Bezugnahme auf die Begriffe "Cycloalkyl", "Cycloalkylalkyl". "Cycloalkenyl", "Cycloalkenylalkyl". "Heterocycloalkyl", "Heterocycloalkenyl", "Heterocycloalkylalkyl" und "Heterocycloalkenylalkyl" bedeutet, daß der cyclische Teil dieser Gruppen durch einen oder mehrere gleiche oder verschiedenartige Reste ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Amino oder Halogenatomen substituiert ist,
- man unter "Aryl" jede mono- oder polycyclische aromatische Gruppe, die 6 bis 22 Kohlenstoffatome enthält, sowie die Biphenylgruppe versteht,
- man unter "Heteroaryl" jede mono- oder polycyclische aromatische Gruppe, die 5 bis 10 Atome aufweist, die 1 bis 3 Heteroatome ausgewählt aus Stickstoff, Sauerstoff oder Schwefel umfassen, versteht,
wobei die "Aryl"- und "Heteroaryl"-gruppen durch eine oder mehrere, gleichartige oder verschiedenartige Reste ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Cyano, Carboxy, Nitro, Amino oder Halogenatomen substituiert sein können.
mit der Maßgabe, daß:
-wenn A einen Indolkern bedeutet, R" ein Wasserstoffatom darstellt,
- wenn A einen Indolkern und R eine Gruppe -NHCOR'ₐ, -NHCOOR'ₐ oder NHCONR'ₐR"ₐ bedeuten, G-R⁵ keine Gruppe -(CH₂)₂-NHCOR_{b} bedeuten kann, in der R_{b} eine (C₁-C₄)-Alkylgruppe oder CF₃-Gruppe darstellt,
-wenn A einen Benzofuran- oder Benzothiophenkern bedeutet, R" von einer Gruppe COPh (worin Ph substituiert oder unsubstituiert ist) verschieden ist,
-wenn A einen Benzofuran- oder Benzothiophenkern bedeutet, R keine Gruppe -NRₐCOR_{c.} -NHSO₂R_{c}. -NHCOCH₂R_{c} oder NHCONHR_{c} bedeuten kann,
worin R_{c} eine heterocyclische Gruppe oder Arylgruppe bedeutet.
- wenn A eine Tetrahydronaphthalingruppe darstellt, R⁵ keine Gruppe CONR'ₐR"ₐ die oben angegebenen Bedeutungen besitzt darstellt,
deren Enantiomere und Diastereoisomere sowie deren Additionssalze mit einer pharmazeutisch annehmbaren Säure oder Base.

2. Verbindungen der Formel (I) nach Anspruch 1, worin R eine Gruppe der Formel (VI) darstellt, in der R³ eine Gruppe COR'ₐ bedeutet, worin R'ₐ die in Anspruch 1 angegebenen Bedeutungen besitzt, deren Enantiomere und Diastereoisomere sowie deren Additionssalze mit einer pharmazeutisch annehmbaren Säure oder Base.

3. Verbindungen der Formel (I) nach Anspruch 1, worin R eine Gruppe der Formel (VI) darstellt, in der R³ eine Gruppe COOR'ₐ bedeutet, worin R'ₐ die in Anspruch 1 angegebenen Bedeutungen besitzt, deren Enantiomere und Diastereoisomere sowie deren Additionssalze mit einer pharmazeutisch annehmbaren Säure oder Base.

4. Verbindungen der Formel (I) nach Anspruch 1, worin R' eine Gruppe G-R⁵ darstellt, worin G eine Alkylenkette -(CH₂)ₜ- bedeutet, worin t den Wert 2 oder 3 besitzt, und R⁵ eine Gruppe 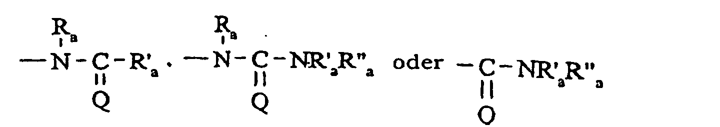 darstellt, in denen Rₐ, R'ₐ, R"ₐ und Q die in Anspruch 1 angegebenen Bedeutungen besitzen, deren Enantiomere und Diastereoisomere sowie deren Additionssalze mit einer pharmazeutisch annehmbaren Säure oder Base.

5. Verbindungen der Formel (I) nach Anspruch 1, worin R' eine Gruppe G-R⁵ darstellt, in der R⁵ eine Gruppe -NHCOR'ₐ oder -CONHR'ₐ darstellt, worin R'ₐ die in Anspruch 1 angegebenen Bedeutungen besitzt, deren Enantiomere und Diastereoisomere sowie deren Additionssalze mit einer pharmazeutisch annehmbaren Säure oder Base.

6. Verbindungen der Formel (I) nach Anspruch 1, worin A ein cyclisches System der Formel (II) bedeutet, deren Enantiomere und Diastereoisomere sowie deren Additionssalze mit einer pharmazeutisch annehmbaren Säure oder Base.

7. Verbindungen der Formel (I) nach Anspruch 1, worin A ein cyclisches System der Formel (III) bedeutet, deren Enantiomere und Diastereoisomere sowie deren Additionssalze mit einer pharmazeutisch annehmbaren Säure oder Base.

8. Verbindungen der Formel (I) nach Anspruch 1, worin A ein cyclisches System der Formel (II) darstellt, R die Gruppe -NHCOR'ₐ darstellt, worin R'ₐ die in Anspruch 1 angegebenen Bedeutungen besitzt und R' eine Gruppe der Formel (IX) darstellt, in der R⁵ eine Gruppe 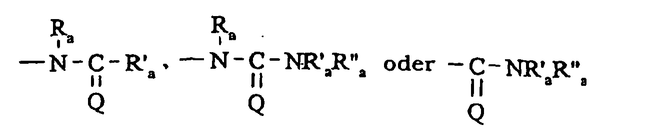 bedeutet, in denen Q, Rₐ, R'ₐ und R"ₐ die in Anspruch 1 angegebenen Bedeutungen besitzen, deren Enantiomere und Diastereoisomere sowie deren Additionssalze mit einer pharmazeutisch annehmbaren Säure oder Base.

9. Verbindungen der Formel (I) nach Anspruch 1, worin A ein cyclisches System der Formel (II) darstellt, R eine Gruppe -NHCOOR'ₐ bedeutet, in der R'ₐ die in Anspruch 1 angegebenen Bedeutungen besitzt und R' eine Gruppe der Formel (IX) darstellt, in der R⁵ eine Gruppe 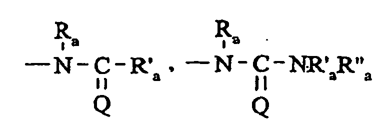 oder 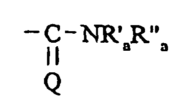 darstellt, worin Q, Rₐ, R'ₐ und R"ₐ die in Anspruch 1 angegebenen Bedeutungen besitzen, deren Enantiomere und Diastereoisomere sowie deren Additionssalze mit einer pharmazeutisch annehmbaren Säure oder Base.

10. Verbindungen der Formel (I) nach Anspruch 1, worin A ein cyclisches System der Formel (III) darstellt, R eine Gruppe -NHCOR'ₐ bedeutet, worin R'ₐ die in Anspruch 1 angegebenen Bedeutungen besitzt und R' eine Gruppe der Formel (IX) darstellt, in der R⁵ eine Gruppe 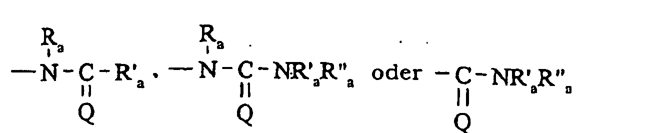 bedeutet, worin Q, Rₐ, R'ₐ und R"ₐ die in Anspruch 1 angegebenen Bedeutungen besitzen, deren Enantiomere und Diastereoisomere sowie deren Additionssalze mit einer pharmazeutisch annehmbaren Säure oder Base.

11. Verbindungen der Formel (I) nach Anspruch 1, worin A ein cyclisches System der Formel (III) bedeutet, R eine Gruppe -NHCOOR'ₐ darstellt, worin R'ₐ die in Anspruch 1 angegebenen Bedeutungen besitzt und R' eine Gruppe der Formel (IX) bedeutet, in der R⁵ eine Gruppe 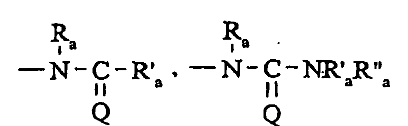 oder 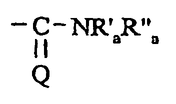 darstellt, worin Q, Rₐ, R'ₐ und R"ₐ die in Anspruch 1 angegebenen
Bedeutungen besitzen, deren Enantiomere und Diastereoisomere sowie deren Additionssalze mit einer pharmazeutisch annehmbaren Säure oder Base.

12. Verbindungen der Formel (I) nach Anspruch 1, wori A einen Naphthalin-, Dihydronaphthalin- oder Tetrahydronaphthalinkern bedeutet, deren Enantiomere und Diastereoisomere sowie deren Additionssalze mit einer pharmazeutisch annehmbaren Säure oder Base.

13. Verbindungen der Formel (I) nach Anspruch 1, worin A einen Benzofuran- oder Dihydrobenzofurankern bedeutet, deren Enantiomere und Diastereoisomere sowie deren Additionssalze mit einer pharmazeutisch annehmbaren Säure oder Base.

14. Verbindungen der Formel (I) nach Anspruch 1, worin A einen Benzothiophen- oder Dihydrobenzothiophenkern bedeutet, deren Enantiomere und Diastereoisomere sowie deren Additionssalze mit einer pharmazeutisch annehmbaren Säure oder Base.

15. Verbindungen der Formel (I) nach Anspruch 1, worin A einen Indol- oder Indolinkern bedeutet, deren Enantiomere und Diastereoisomere sowie deren Additionssalze mit einer pharmazeutisch annehmbaren Säure oder Base.

16. Verbindungen der Formel (I) nach Anspruch 1, worin A einen Naphthalin-, Dihydronaphthalin- oder Tetrahydronaphthalinkern bedeutet, R eine Gruppe -NHCORₐ darstellt, worin Rₐ die in Anspruch 1 angegebenen Bedeutungen besitzt und R' eine Gruppe -(CH₂)ₜ-NHCOR'ₐ oder -(CH₂)ₜ-CONHR'ₐ bedeutet, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

17. Verbindungen der Formel (I) nach Anspruch 1, worin A einen Benzofuran- oder Dihydrobenzofurankern bedeutet, R eine Gruppe -NHCORₐ darstellt, worin Rₐ die in Anspruch 1 angegebenen Bedeutungen besitzt und R' eine Gruppe -(CH₂)ₜ-NHCOR'ₐ oder -(CH₂)ₜ-CONHR'ₐ darstellt, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

18. Verbindungen der Formel (I) nach Anspruch 1, worin A einen Benzothiophen- oder Dihydrobenzothiophenkern bedeutet, R eine Gruppe -NHCORₐ darstellt, worin Rₐ die in Anspruch 1 angegebenen Bedeutungen besitzt und R' eine Gruppe -(CH₂)ₜ-NHCOR'ₐ oder -(CH₂)ₜ-CONHR'ₐ darstellt, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

19. Verbindungen der Formel (I) nach Anspruch 1, worin A einen Indol- oder Indolinkern bedeutet, R eine Gruppe -NHCORₐ darstellt, worin Rₐ die in Anspruch 1 angegebenen Bedeutungen besitzt und R_{'} eine Gruppe -(CH₂)ₜ-NHCOR'ₐ oder -(CH₂)ₜ-CONHR'ₐ darstellt, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

20. Verbindung der Formel (I) nach Anspruch 1, worin A einen Naphthalin-, Dihydronaphthalin- oder Tetrahydronaphthalinkern bedeutet, R eine Gruppe -NHCOORₐ oder -N(alk)COORₐ darstellt, worin Rₐ die in Anspruch 1 angegebenen Bedeutungen besitzt und alk eine Alkylgruppe darstellt, und R' eine Gruppe -(CH₂)ₜ-NHCOR_{'a} oder -(CH₂)ₜ-CONHR'ₐ darstellt, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

21. Verbindungen der Formel (I) nach Anspruch 1, worin A einen Benzofuran- oder Dihydrobenzofurankern bedeutet, R eine Gruppe -NHCOORₐ oder -N(alk)COORₐ darstellt, worin Rₐ die in Anspruch 1 angegebenen Bedeutungen besitzt und alk eine Alkylgruppe darsetllt, und R' eine Gruppe -(CH₂)ₜ-NHCOR'ₐ oder -(CH₂)ₜ-CONHR'ₐ bedeutet, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

22. Verbindungen der Formel (I) nach Anspruch 1, worin A einen Benzothiophen- oder Dihydrobenzothiophenkern bedeutet, R eine Gruppe -NHCOORₐ oder -N(alk)COORₐ darstellt, worin Rₐ die in Anspruch 1 angegebenen Bedeutungen besitzt und alk eine Alkylgruppe darstellt, und R' eine Gruppe -(CH₂)ₜ-NHCOR'ₐ oder -(CH2)ₜ-CONHR'ₐ bedeutet, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

23. Verbindungen der Formel (I) nach Anspruch 1, worin A einen Indol- oder Indolinkern bedeutet, R eine Gruppe -NHCOORₐ oder -N(alk)COORₐ darstellt, worin Rₐ die in Anspruch 1 angegebenen Bedeutungen besitzt und alk eine Alkylgruppe darstellt, und R' eine Gruppe -(CH₂)ₜ-NHCOR'ₐ oder -(CH₂)ₜ-CONHR'ₐ bedeutet, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

24. Verbindungen der Formel (I) nach Anspruch 1, nämlich:
* N-{3-[2-(Acetylamino)-ethyl]-1-benzofuran-5-yl}-2,2,2-trifluoracetamid,
* N-{2-[5-(Acetylamino)-1-benzofuran-3-yl]-ethyl}-cyclopropancarboxamid,
* N-{2-[5-(Acetylamino)-1-benzothiophen-3-yl]-ethyl}-benzamid,
deren Enantiomere und Diastereoisomere sowie deren Additionssalze mit einer pharmazeutisch annehmbaren Säure oder Base.

25. Verbindungen der Formel (I) nach Anspruch 1, nämlich:
* N-{8-[2-([2-Phenylacetyl]-amino)-ethyl]-2-naphthyl}-butanamid,
* N-(8-{2-[(2-Bromacetyl)-amio]-ethyl}-2-naphthyl)-1-cyclohexan-carboxamid.
* N-{8-[2-(Heptanoylamino)-ethyl]-2,6-dinaphthyl}-2-butenamid,
* N-{8-[2-(Acetylamino)-ethyl]-2-naphthyl}-acetamid,
deren Enantiomere und Diastereoisomere sowie deren Additionssalze mit einer pharmazeutisch annehmbaren Säure oder Base.

26. Verbindungen der Formel (I) nach Anspruch 1, nämlich:
* N-{3-[2-(Acetylamino)-ethyl]-benzo[*b*]furan-5-yl}-carbamidsäure-methylester,
* 3-{2-[(Cyclopropylcarbonyl)-amino]-ethyl}-1-benzofuran-1-yl-carbamidsäure-methylester,
* 3-[2-(Acetylamino)-ethyl]-1-benzofuran-5-yl-carbamidsäure-tert.-butylester,
* 3-[2-(Acetylamino)-ethyl]-1-benzofuran-5-yl-(methyl)carbamidsäure-tert.-butylester,
* 3-[2-(Benzoylamino)-ethyl]-1-benzofuran-5-yl-carbamidsäure-methylester,
* 3-[2-(Isobutyrylamino)-ethyl]-1-benzofuran-5-yl-carbamidsäure-methylester,
* 3-[2-(2-Furoylamino)-ethyl]-1-benzofuran-5-yl-carbamidsäure-methylester,
* 3-{2- [(Cyclopentylcarbonyl)-amino]-ethyl}-1- benzofuran-5-yl-carbamidsäure-methylester,
deren Enantiomere und Diastereoisomere sowie deren Additionssalze mit einer pharmazeutisch annehmbaren Säure oder Base.

27. Verbindungen der Formel (I) nach Anspruch 1, nämlich:
* N-(8-{2-[(2-Bromacetyl)-amino]-ethyl}-2-naphthyl)-carbamidsäure-ethylester,
* N-{8-[2-(Acetylamino)-ethyl]-6-phenyl-2-naphthyl}-carbamidsäure-methylester,
* N-{8-[2-(Acetylamino)-ethyl]-5,6,7,8-tetrahydro-2-naphthalenyl}-carbamidsäure-hexylester,
* 8-[2-(Acetylamino)-ethyl]-2-naphthyl-carbamidsäure-methylester,
deren Enantiomere und Diastereoisomere sowie deren Additionssalze mit einer pharmazeutisch annehmbaren Säure oder Base.

28. Verfahren zur Herstellung der Verbindungen der Formel (I), **dadurch gekennzeichnet, daß** man als Ausgangsprodukt die Verbindung der Formel (X) verwendet: 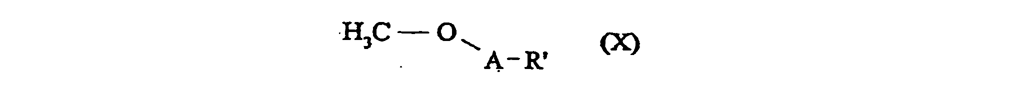 in der A und R' die in Anspruch 1 angegebenen Bedeutungen besitzen, welche man einer Demethylierung unterwirft unter Verwendung klassischer Mittel, wie HBr, AlCl₃, AlBr₃, BBr₃ oder binären Systemen aus Lewis-Säuren und nucleophilen Mitteln, wie beispielsweise AlCl₃/PhCH₂SH der BBr₃/Me₂S, zur Bildung der Verbindung der Formel (XI):
HO - A - R' (XI)
in der A und R' die oben angegebenen Bedeutungen besitzen,
welche man unter der Einwirkung von Reagenzien, wie POCl₃, PCl₅, Ph₃PBr₂, PhPCl₄, HBr oder HI in das entsprechende Halogenderivat der Formel (XII) umwandelt:
Hal - A - R' (XII)
in der A und R' die oben angegebenen Bedeutungen besitzen und Hal ein Halogenatom darstellt (wobei die Verbindungen der Formel (XII) durch Austauschreaktionen erhalten werden können, beispielsweise durch Behandeln eines Chlorderivats mit KF in Dimethylformamid zur Bildung des entsprechenden Fluorderivats, oder durch Behandeln eines Bromderivats mit KI in Gegenwart von Kupfersalzen zur Bildung des entsprechenden Iodderivats),
welches mit:
Kohlenmonoxid und Bu₃SnH behandelt wird, wobei die Reaktion durch Palladium(0) katalysiert wird, zur Bildung des entsprechenden Aldehyds der Formel (XIII) : 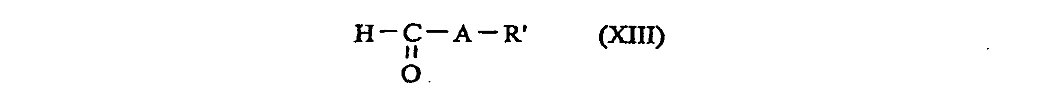 in der A und R' die oben angegebenen Bedeutungen besitzen,
welche Verbindung der Formel (XIII) auch erhalten werden kann ausgehend von dem Halogenderivat der Formel (XII) unter Anwendung klassischer Lithierungsmethoden, oder über das entsprechende Vinylderivat (welches ausgehend von der Verbindung der Formel (XII) durch Einwirkung von Vinyltributylzinn und Palladiumtetrakis erhalten worden ist), welches einer Ozonolyse unterworfen wird, oder durch direkte Formylierung des Kerns A mit Hilfe beispielsweise einer Vilsmeier-Reaktion,
welche Verbindung der Formel (XIII) der Einwirkung eines Oxidationsmittels unterworfen wird zur Bildung der Verbindung der Formel (XIV):
HOOC - A - R' (XIV)
in der A und R' die oben angegebenen Bedeutungen besitzen, welche: nach der Einwirkung von Thionylchlorid und einem Azid und dann einer Säure in die Verbindung der Formel (XV) umgewandelt wird:
HₐN-A-R' (XV)
in der A und R' die oben angegebenen Bedeutungen besitzen, welche man:
- entweder mit einem Acylchlorid ClCORₐ oder dem entsprechenden (gemischten oder symmetrischen) Anhydrid, worin Rₐ die oben angegebenen Bedeutungen besitzt, kondensiert zur Bildung der Verbindung der Formel (I/c), einem Sonderfall der Verbindungen der Formel (I): 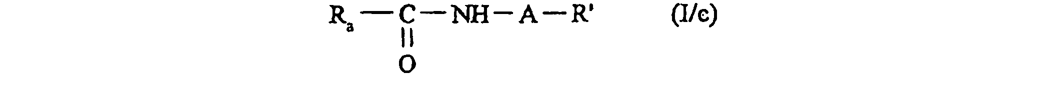 in der Rₐ, A und R' die oben angegebenen Bedeutungen besitzen, welche der Einwirkung einer Verbindung der Formel (XVI) unterworfen werden kann:
R¹ ₐ - J (XVI)
in der R¹ₐ jede Bedeutung von Rₐ aufweisen kann mit Ausnahme des Wasserstoffatoms und J eine austretende Gruppe, wie ein Halogenatom oder eine Tosylgruppe, darstellt,
zur Bildung der Verbindung der Formel (I/d), einem Sonderfall der Verbindungen der Formel (I): 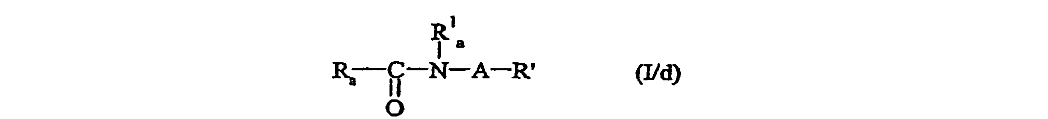 in der Rₐ, R¹ₐ, A und R' die oben angegebenen Bedeutungen besitzen,
welche Verbindungen der Formeln (I/c) und (I/d) die Verbindung der Formel (I/e) bilden, einem Sonderfall der Verbindungen der Formel (I): 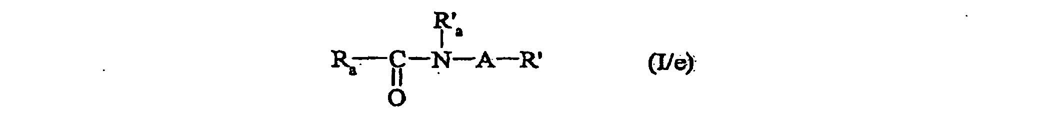 in der Rₐ, R'ₐ. A und R' die oben angegebenen Bedeutungen besitzen,
welche Verbindung der Formel (I/e) man der Einwirkung eines Thionierungsmittels, wie beispielsweise dem Lawesson-Reagens, unterwerfen kann, zur Bildung der Verbindung der Formel (I/f), einem Sonderfall der Verbindungen der Formel (I) : 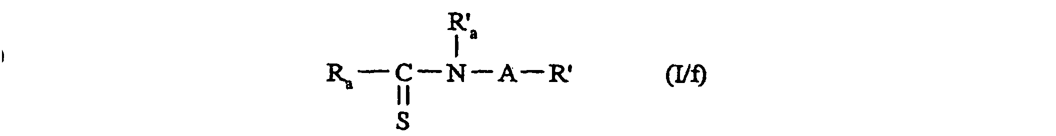 in der Rₐ, R'ₐ, A und R' die oben angegebenen Bedeutungen besitzen,
- oder mit einer Verbindung der Formel (XVII) kondensiert:
Q = C = N - R' ₐ (XVII)
in der Q und R'ₐ die oben angegebenen Bedeutungen besitzen,
zur Bildung der Verbindung der Formel (I/g), einem Sonderfall der Verbindungen der Formel (I): 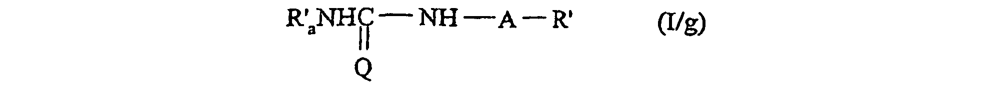 in der R'ₐ, Q, A und R' die oben angegebenen Bedeutungen besitzen,
welche der Einwirkung einer Verbindung der Formel (XVI) unterworfen werden kann, zur Bildung der Verbindung der Formel (I/h), einem Sonderfall der Verbindungen der Formel (I): 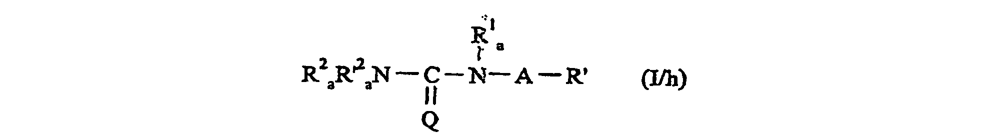 in der Q, R¹ₐ, A und R' die oben angegebenen Bedeutungen besitzen und R²ₐ und R'²ₐ, die gleichartig oder verschieden sein können, sämtliche Bedeutungen von Rₐ annehmen können, mit Ausnahme des Wasserstoffatoms, und die nicht zusammen mit dem sie tragenden Stickstoffatom eine cyclische Struktur bilden können,
- oder mit einer Verbindung der Formel (XVIII): 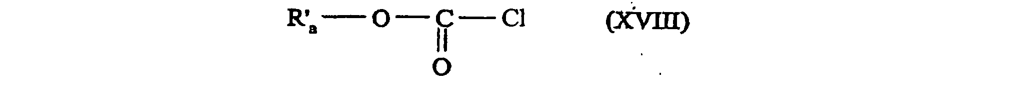 in der R'ₐ die oben angegebenen Bedeutungen besitzt, oder seinem entsprechenden Anhydrid (R'ₐOCO)₂O kondensiert.
zur Bildung der Verbindung der Formel (I/i), einem Sonderfall der Verbindungen der Formel (I): 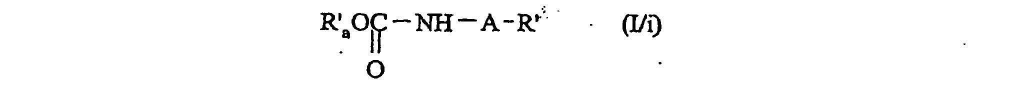 in der R'ₐ, A und R' die oben angegebenen Bedeutungen besitzen,
welche der Einwirkung einer Verbindung der Formel (XVI) und/oder der Einwirkung eines Thionierungsmittels unterworfen werden kann zur Bildung der Verbindung der Formel (I/j), einem Sonderfall der Verbindungen der Formel (I): 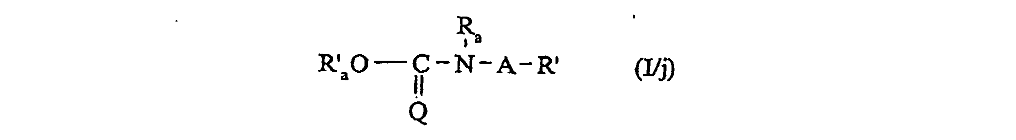 in der Rₐ, R'ₐ, Q, A und R' die oben angegebenen Bedeutungen besitzen,
- oder mit einer Verbindung der Formel (XIX):
RₐSO₂Cl (XIX)
in der Rₐ die oben angegebenen Bedeutungen besitzt, kondensiert, gegebenenfalls gefolgt von der Einwirkung einer Verbindung der Formel (XVI) zur Bildung der Verbindung der Formel (I/k), einem Sonderfall der Verbindungen der Formel (I): 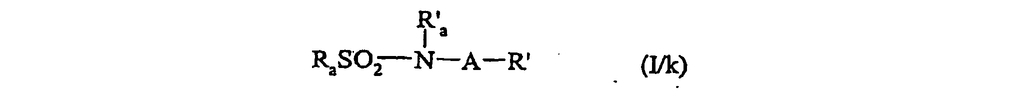 in der Rₐ, R'ₐ, A und R die oben angegebenen Bedeutungen besitzen,
welche Verbindungen (I/c) bis (I/k) mit Hilfe einer klassischen Trennmethode gereinigt werden können, welche man gewünschtenfalls in ihre Additionssalze mit einer pharmazeutisch annehmbaren Säure oder Base überführt und welche man gegebenenfalls mit Hilfe einer klassischen Trennmethode in ihre Isomeren auftrennt.

29. Verfahren zur Herstellung der Verbindungen der Formel (I) nach Anspruch 1, **dadurch gekennzeichnet, daß** man als Ausgangsprodukt die Verbindung der Formel (XXII) verwendet: 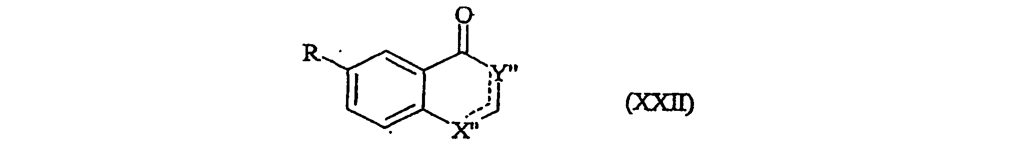 in der R und das Symbol .... die in Anspruch 1 angegebenen Bedeutungen besitzen, Y" eine Bindung darstellt und in diesem Fall X" ein Sauerstoff- oder Schwefelatom oder eine Gruppe NRₒ bedeutet (worin Rₒ die oben angegebenen Bedeutungen besitzt) oder X" und Y", die gleich oder verschiedenartig sind, eine Gruppe C(H)_{q} (worin q 1 oder 2 bedeutet) bedeuten,
welche man einer Wittig-Reaktion und dann einer Reduktion unterzieht zur Bildung der Verbindung der Formel (XXIII): 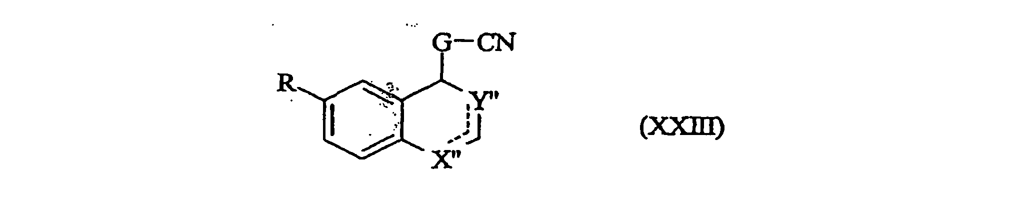 in der R, X", Y", G und das Symbol .... die oben angegebenen Bedeutungen besitzen,
welche oxidiert werden kann zur Bildung der Verbindung der Formel (XXIV): 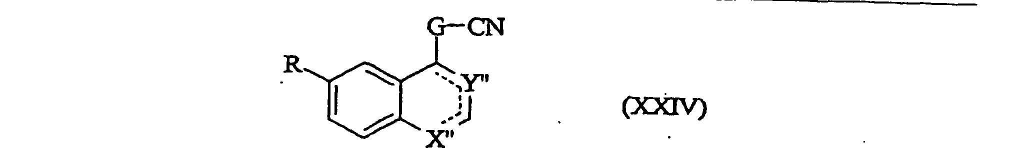 in der R, X", Y", G und das Symbol .... die oben angegebenen Bedeutungen besitzen,
welche
* entweder in saurem oder basischem Medium hydrolysiert wird und dann nach der Aktivierung in Form des Säurechlorids oder in Gegenwart eines Kupplungsmittels der Einwirkung eines Amins HNR'ₐR"ₐ, worin R'ₐ und R"ₐ die oben angegebenen Bedeutungen besitzen, unterworfen wird zur Bildung der Verbindung der Formel (I/p), einem Sonderfall der Verbindungen der Formel (I) : 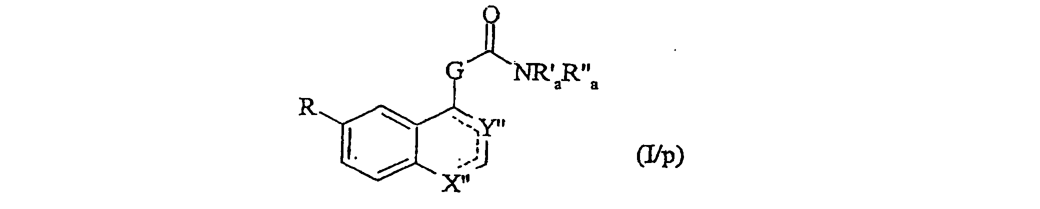 in der R, X", Y", G, R'ₐ, R"ₐ und das Symbol .... die oben angegebenen Bedeutungen besitzen,
welche man der Einwirkung eines Thionierungsmittels, wie dem Lawesson-Reagens, unterwerfen kann, zur Bildung der Verbindung der Formel (I/q), einem Sonderfall der Verbindungen der Formel (I): 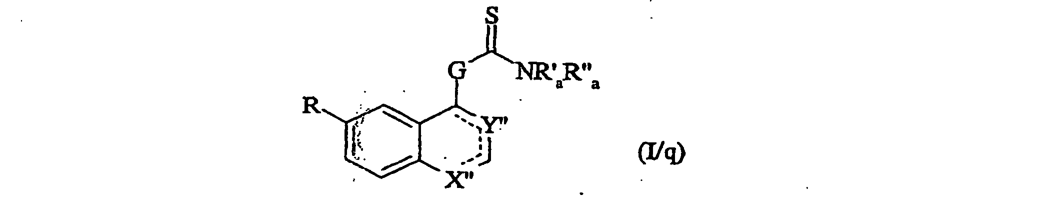 in der R, X", Y", G, R'ₐ, R"ₐ und das Symbol .... die oben angegebenen Bedeutungen besitzen,
* oder in saurem oder basischem Medium hydrolysiert und dann in das entsprechende Azid überführt wird, so daß man nach der Durchführung einer Curtius-Umlagerung und einer Hydrolyse die Verbindung der Formel (XXV) erhält: 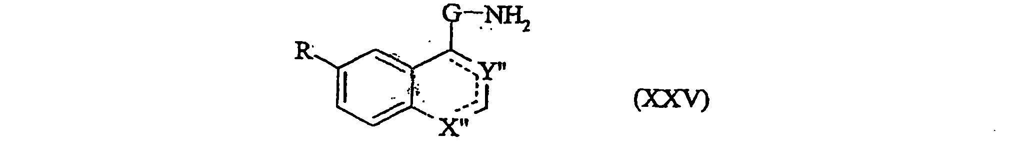 in der R, X", Y" und G die oben angegebenen Bedeutungen besitzen,
welche:
- mit einem Acylchlorid ClCOR'ₐ oder dem entsprechenden (gemischten oder symmetrischen) Anhydrid, worin R'ₐ die oben angegebenen Bedeutungen besitzt, umgesetzt wird gegebenenfalls gefolgt von der Einwirkung einer Verbindung der Formel (XVI) und/oder der Einwirkung eines Thionierungsmittels, so daß man die Verbindung der Formel (I/r) erhält, einem Sonderfall der Verbindungen der Formel (I): 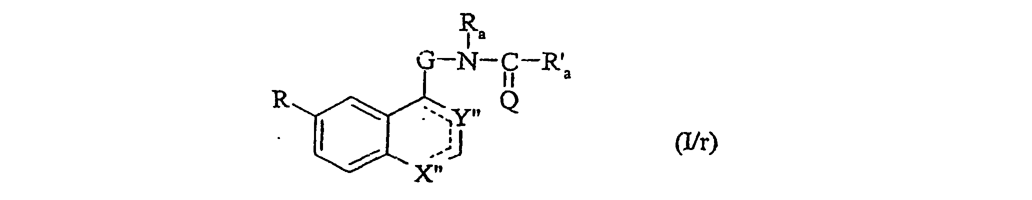 in der R, X", Y", G, Rₐ, R'ₐ, Q und das Symbol .... die oben angegebenen Bedeutungen besitzen,
- oder mit der Verbindung der Formel (XVII) umgesetzt wird, gegebenenfalls gefolgt von der Einwirkung einer Verbindung der Formel (XVI), so daß man schließlich die Verbindung der Formel (I/s) erhält, einem Sonderfall der Verbindungen der Formel (I): 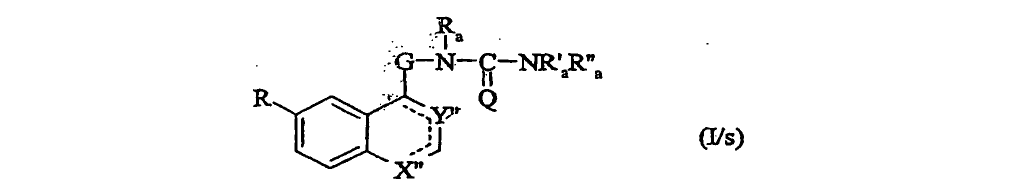 in der R, X", Y", G, Rₐ, R'ₐ, R"ₐ, Q und das Symbol .... die oben angegebenen Bedeutungen besitzen,
welche Verbindungen (I/p) bis (I/s) mit Hilfe einer klassischen Trennmethode gereinigt werden können, welche man gewünschtenfalls in ihre Additionssalze mit einer pharmazeutisch annehmbaren Säure oder Base überführt und welche man gegebenenfalls mit Hilfe einer klassischen Trennmethode in ihre Isomeren auftrennt.

30. Pharmazeutische Zubereitungen enthaltend die Produkte der Formel (I) nach einem der Ansprüche 1 bis 27 oder eines ihrer Additionssalze mit einer pharmazeutisch annehmbaren Säure oder Base in Kombination mit einem oder mehreren pharmazeutisch annehmbaren Hilfsstoffen.

31. Pharmazeutische Zubereitungen nach Anspruch 30 für die Herstellung von Arzneimitteln für die Behandlung von Störungen, die mit dem melatoninergischen System verknüpft sind.

## Claims

1. Compounds of formula (I) :
R-A-R' (I)
wherein :
◆ A represents :
― a ring system of formula (II) : 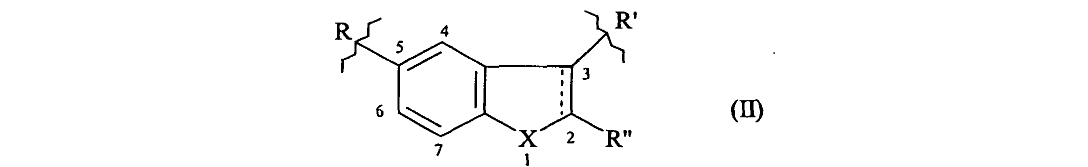 wherein
• X represents an oxygen or sulphur atom or a group NR₀ (wherein R₀ represents a hydrogen atom, a linear or branched (C₁-C₆)alkyl group, an aryl group, an aryl-(C₁-C₆)alkyl group in which the alkyl moiety is linear or branched, or SO₂Ph),
• the symbol ..... means that the bond may be single or double, it being understood that the valency of the atoms is respected,
• R" represents a Rₐ, ORₐ, CORₐ, COORₐ, OCORₐ, OSO₂CF₃ group or halogen atoms,
wherein Rₐ represents a hydrogen atom, an unsubstituted or substituted linear or branched (C₁-C₆)alkyl group, an unsubstituted or substituted linear or branched (C₂-C₆)alkenyl group, an unsubstituted or substituted linear or branched (C₂-C₆)-alkynyl group, a linear or branched (C₁-C₆)polyhaloalkyl group, an unsubstituted or substituted (C₃-C₈)cycloallcyl group, an unsubstituted or substituted (C₃-C₈)-cycloalkyl-(C₁-C₆)alkyl group in which the alkyl moiety is linear or branched, an unsubstituted or substituted (C₃-C₈)cycloalkenyl group, an unsubstituted or substituted (C₃-C₈)cycloalkenyl-(C₁-C₆)alkyl group in which the alkyl moiety is linear or branched, an aryl group, an aryl-(C₁-C₆)alkyl group in which the alkyl moiety is linear or branched, an aryl-(C₁-C₆)alkenyl group in which the alkenyl moiety is linear or branched, a heteroaryl group, a heteroaryl-(C₁-C₆)alkyl group in which the alkyl moiety is linear or branched, a heteroaryl-(C₁-C₆)alkenyl group in which the alkenyl moiety is linear or branched, an unsubstituted or substituted heterocycloalkyl group, an unsubstituted or substituted heterocycloalkenyl group, an unsubstituted or substituted heterocycloalkyl-(C₁-C₆)alkyl group in which the alkyl moiety is linear or branched, or an unsubstituted or substituted heterocycloalkenyl-(C₁-C₆)alkyl group in which the alkyl moiety is linear or branched,
― or a ring system of formula (III) : 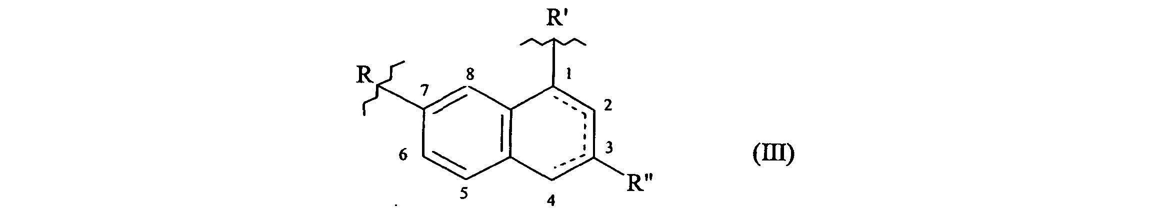 wherein R" and the symbol ..... are as defined hereinbefore,
◆ R represents :
― a group of formula (VI) : 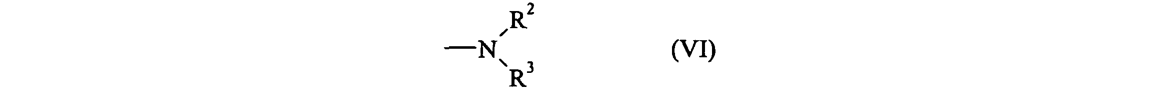 wherein
• R² represents a group Rₐ as defined hereinbefore,
• R³ represents a group COR'ₐ, CSR'ₐ, CONR'ₐR"ₐ, CSNR'ₐR"ₐ, COOR'ₐ, CSOR'ₐ or S(O)ᵥR'ₐ (wherein R'ₐ and R"ₐ, which may be the same or different, can take all the meanings of Rₐ as defined hereinbefore and may also form, together with the nitrogen atom carrying them, a cyclic group which may contain, in addition to the nitrogen atom by which it is linked, from one to three heteroatoms selected from oxygen, sulphur and nitrogen, and v is 1 or 2),
◆ and R' represents a group of formula (IX) :
―G ―R⁵ (IX)
wherein
• G represents an alkylene chain -(CH₂)ₜ- (wherein t is 2 or 3),
• and R⁵ represents a group 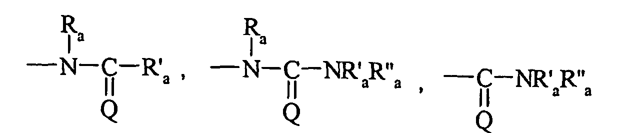 or 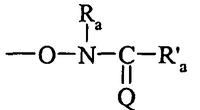 wherein Q represents an oxygen or a sulphur atom, and Rₐ, R'ₐ and R"ₐ (which may be the same or different) are as defined hereinbefore, it being possible for R'ₐ and R"ₐ to form, together with the nitrogen atom carrying them, a cyclic group as defined hereinbefore,
it being understood that:
― "heterocycloalkyl" is taken to mean any saturated mono- or poly-cyclic group containing from 5 to 10 atoms including from 1 to 3 hetero atoms selected from nitrogen, oxygen and sulphur,
― "heterocycloalkenyl" is taken to mean any non-aromatic mono- or poly-cyclic group containing one or more unsaturated bonds, containing from 5 to 10 atoms and which may contain from 1 to 3 hetero atoms selected from nitrogen, oxygen and sulphur,
― the term "substituted" used in respect of the expressions "alkyl", "alkenyl" and "alkynyl" indicates that the groups in question are substituted by one or more radicals, which may be the same or different, selected from hydroxy, linear or branched (C₁-C₆)alkoxy, linear or branched (C₁-C₆)alkyl, linear or branched (C₁-C₆)polyhaloalkyl, amino and halogen atoms,
― the term "substituted" used in respect of the expressions "cycloalkyl", "cycloalkylalkyl", "cycloalkenyl", "cycloalkenylalkyl", "heterocycloalkyl", "heterocycloalkenyl", "heterocycloalkylalkyl" and "heterocycloalkenylalkyl" indicates that the cyclic moiety of the groups in question is substituted by one or more radicals, which may be the same or different, selected from hydroxy, linear or branched (C₁-C₆)alkoxy, linear or branched (C₁-C₆)alkyl, linear or branched (C₁-C₆)polyhaloalkyl, amino and halogen atoms,
― "aryl" is taken to mean any aromatic, mono- or poly-cyclic group containing from 6 to 22 carbon atoms, and also the biphenyl group,
― "heteroaryl" is taken to mean any aromatic mono- or poly-cyclic group containing from 5 to 10 atoms containing from 1 to 3 hetero atoms selected from nitrogen, oxygen and sulphur,
it being possible for the "aryl" and "heteroaryl" groups to be substituted by one or more radicals, which may be the same or different, selected from hydroxy, linear or branched (C₁-C₆)alkoxy, linear or branched (C₁-C₆)alkyl, linear or branched (C₁-C₆)polyhaloalkyl, cyano, carboxy, nitro, amino and halogen atoms,
it being understood that :
― when A represents an indole nucleus, R" represents a hydrogen atom,
― when A represents an indole nucleus and R represents a group NHCOR'ₐ, -NHCOOR'ₐ or NHCONR'ₐR"ₐ, then G-R⁵ cannot represent a group -(CH₂)₂-NHCOR_{b} wherein R_{b} represents a (C₁-C₄)alkyl or CF₃ group,
― when A represents a benzofuran or benzothiophene nucleus, R" is other than a COPh groups (Ph being substituted or unsubstituted),
― when A represents a benzofuran or benzothiophene nucleus, R cannot represent a group -NRₐCOR_{c}, -NHSO₂R_{c}, -NHCOCH₂R_{c} or NHCONHR_{c} wherein R_{c} represents a heterocyclic or aryl group,
― when A represents a tetrahydronaphthalene group, R⁵ cannot represent a group CONR'ₐR"ₐ as defined hereinbefore,
their enantiomers and diastereoisomers, and addition salts thereof with a pharmaceutically acceptable acid or base.

2. Compounds of formula (I) according to claim 1, wherein R represents a group of formula (VI) wherein R³ represents a group COR'ₐ wherein R'ₐ is as defined in claim 1, their : enantiomers and diastereoisomers, and addition salts thereof with a pharmaceutically acceptable acid or base.

3. Compounds of formula (I) according to claim 1, wherein R represents a group of formula (VI) wherein R³ represents a group COOR'ₐ wherein R'ₐ is as defined in claim 1, their enantiomers and diastereoisomers, and addition salts thereof with a pharmaceutically acceptable acid or base.

4. Compounds of formula (I) according to claim 1, wherein R' represents a group G-R⁵ wherein G represents an alkylene chain -(CH₂)ₜ- wherein t is 2 or 3 and R⁵ represents a group 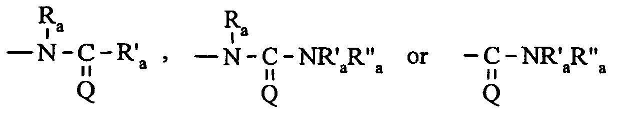 wherein Rₐ, R'ₐ, R"ₐ and Q are as defined in claim 1, their enantiomers and diastereoisomers, and addition salts thereof with a pharmaceutically acceptable acid or base.

5. Compounds of formula (I) according to claim 1, wherein R' represents a group G-R⁵ wherein R⁵ represents a group NHCOR'ₐ or -CONHR'ₐ wherein R'ₐ is as defined in claim 1, their enantiomers and diastereoisomers, and addition salts thereof with a pharmaceutically acceptable acid or base.

6. Compounds of formula (I) according to claim 1, wherein A represents a ring system of formula (II), their enantiomers and diastereoisomers, and addition salts thereof with a pharmaceutically acceptable acid or base.

7. Compounds of formula (I) according to claim 1, wherein A represents a ring system of formula (III), their enantiomers and diastereoisomers, and addition salts thereof with a pharmaceutically acceptable acid or base.

8. Compounds of formula (I) according to claim 1, wherein A represents a ring system of formula (II), R represents a group -NHCOR'ₐ wherein R'ₐ is as defined in claim 1, and R' represents a group of formula (IX) wherein R⁵ represents a group 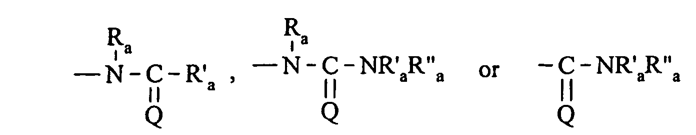 wherein Q, Rₐ, R'ₐ and R"ₐ are as defined in claim 1, their enantiomers and diastereoisomers, and addition salts thereof with a pharmaceutically acceptable acid or base.

9. Compounds of formula (I) according to claim 1, wherein A represents a ring system of formula (II), R represents a group NHCOOR'ₐ wherein R'ₐ is as defined in claim 1, and R' represents a group of formula (IX) wherein R⁵ represents a group 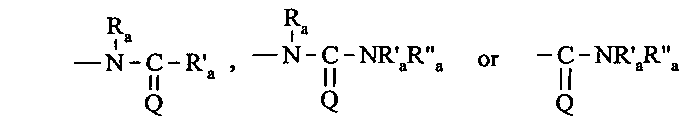 wherein Q, Rₐ, R'ₐ and R"ₐ are as defined in claim 1, their enantiomers and diastereoisomers, and addition salts thereof with a pharmaceutically acceptable acid or base.

10. Compounds of formula (I) according to claim 1, wherein A represents a ring system of formula (III), R represents a group NHCOR'ₐ wherein R'ₐ is as defined in claim 1, and R' represents a group of formula (IX) wherein R⁵ represents a group 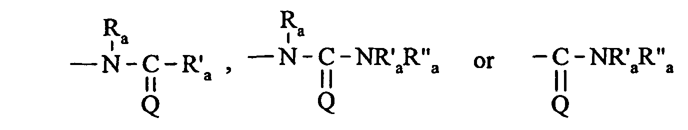 wherein Q, Rₐ, R'ₐ and R"ₐ are as defined in claim 1, their enantiomers and diastereoisomers, and addition salts thereof with a pharmaceutically acceptable acid or base.

11. Compounds of formula (I) according to claim 1, wherein A represents a ring system of formula (III), R represents a group -NHCOOR'ₐ wherein R'ₐ is as defined in claim 1, and R' represents a group of formula (IX) wherein R⁵ represents a group 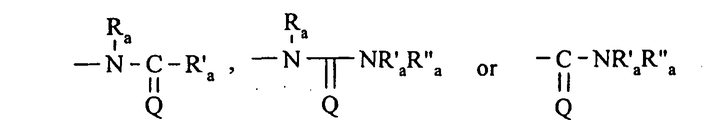 wherein Q, Rₐ, R'ₐ and R"ₐ are as defined in claim 1, their enantiomers and diastereoisomers, and addition salts thereof with a pharmaceutically acceptable acid or base.

12. Compounds of formula (I) according to claim 1, wherein A represents a naphthalene, dihydro- or tetrahydro-naphthalene nucleus, their enantiomers and diastereoisomers, and addition salts thereof with a pharmaceutically acceptable acid or base.

13. Compounds of formula (I) according to claim 1, wherein A represents a benzofuran or dihydrobenzofuran nucleus, their enantiomers and diastereoisomers, and addition salts thereof with a pharmaceutically acceptable acid or base.

14. Compounds of formula (I) according to claim 1, wherein A represents a benzothiophene or dihydrobenzothiophene nucleus, their enantiomers and diastereoisomers, and addition salts thereof with a pharmaceutically acceptable acid or base.

15. Compounds of formula (I) according to claim 1, wherein A represents an indole or indoline nucleus, their enantiomers and diastereoisomers, and addition salts thereof with a pharmaceutically acceptable acid or base.

16. Compounds of formula (I) according to claim 1, wherein A represents a naphthalene, dihydro- or tetrahydro-naphthalene nucleus, R represents a group -NHCORₐ, wherein Rₐ is as defined in claim 1, and R' represents a group -(CH₂)ₜ-NHCOR'ₐ or -(CH₂),-CONHR'ₐ, wherein t is 2 or 3 and R'ₐ is as defined in claim 1, their enantiomers and diastereoisomers, and addition salts thereof with a pharmaceutically acceptable acid or base.

17. Compounds of formula (I) according to claim 1, wherein A represents a benzofuran or dihydrobenzofuran nucleus, R represents a group -NHCORₐ, wherein Rₐ is as defined in claim 1, and R' represents a group -(CH₂)ₜ-NHCOR'ₐ or -(CH₂)ₜ-CONHR'ₐ, wherein t is 2 or 3 and R'ₐ is as defined in claim 1, their enantiomers and diastereoisomers, and addition salts thereof with a pharmaceutically acceptable acid or base.

18. Compounds of formula (I) according to claim 1, wherein A represents a benzothiophene or dihydrobenzothiophene nucleus, R represents a group -NHCORₐ, wherein Rₐ is as defined in claim 1, and R' represents a group -(CH₂)ₜ-NHCOR'ₐ or -(CH₂)ₜ-CONHR'ₐ, wherein t is 2 or 3 and R'ₐ is as defined in claim 1, their enantiomers and diastereoisomers, and addition salts thereof with a pharmaceutically acceptable acid or base.

19. Compounds of formula (I) according to claim 1, wherein A represents an indole or indoline nucleus, R represents a group -NHCORₐ, wherein Rₐ is as defined in claim 1, and R' represents a group -(CH₂)ₜ-NHCOR'ₐ or -(CH₂)ₜCONHR'ₐ, wherein t is 2 or 3 and R'ₐ is as defined in claim 1, their enantiomers and diastereoisomers, and addition salts thereof with a pharmaceutically acceptable acid or base.

20. Compound of formula (I) according to claim 1, wherein A represents a naphthalene, dihydronaphthalene or tetrahydronaphthalene nucleus, R represents a group -NHCOORₐ or -N(alk)COORₐ wherein Rₐ is as defined in claim 1 and alk represents an alkyl group, and R' represents a group -(CH₂)ₜ-NHCOR'ₐ or -(CH₂)ₜ-CONHR'ₐ, wherein t is 2 or 3 and R'ₐ is as defined in claim 1, their enantiomers and diastereoisomers, and addition salts thereof with a pharmaceutically acceptable acid or base.

21. Compounds of formula (I) according to claim 1, wherein A represents a benzofuran or dihydrobenzofuran nucleus, R represents -NHCOORₐ or -N(alk)COORₐ wherein Rₐ is as defined in claim 1 and alk represents an alkyl group, and R' represents a group -(CH₂)ₜ-NHCOR'ₐ or -(CH₂)ₜ-CONHR'ₐ, wherein t is 2 or 3 and R'ₐ is as defined in claim 1, their enantiomers and diastereoisomers, and addition salts thereof with a pharmaceutically acceptable acid or base.

22. Compounds of formula (I) according to claim 1, wherein A represents a benzothiophene or dihydrobenzothiophene nucleus, R represents a group -NHCOORₐ or -N(alk)COORₐ wherein Rₐ is as defined in claim 1 and alk represents an alkyl group, and R' represents a group -(CH₂)ₜ-NHCOR'ₐ or -(CH₂)ₜ-CONHR'ₐ, wherein t is 2 or 3 and R'ₐ is as defined in claim 1, their enantiomers and diastereoisomers, and addition salts thereof with a pharmaceutically acceptable acid or base.

23. Compounds of formula (I) according to claim 1, wherein A represents an indole or indoline nucleus, R represents a group -NHCOORₐ or -N(alk)COORₐ wherein Rₐ is as defined in claim 1 and alk represents an alkyl group, and R' represents a group -(CH₂)ₜ-NHCOR'ₐ or -(CH₂)ₜ-CONHR'ₐ, wherein t is 2 or 3 and R'ₐ is as defined in claim 1, their enantiomers and diastereoisomers, and addition salts thereof with a pharmaceutically acceptable acid or base.

24. Compounds of formula (I) according to claim 1 that are :
* N-{3-[2-(acetylamino)ethyl]-1-benzofuran-5-yl}-2,2,2-trifluoroacetamide,
* N-{2-[5-(acetylamino)-1-benzofuran-3-yl]ethyl}cyclopropanecarboxamide,
* N-{2-[5-(acetylamino)-1-benzothiophen-3-yl]ethyl}benzamide,
their enantiomers and diastereoisomers, and addition salts thereof with a pharmaceutically acceptable acid or base.

25. Compounds of formula (I) according to claim 1 that are :
* N-{8-[2-([2-phenylacetyl]amino)ethyl]-2-naphthyl}butanamide,
* N-(8-{2-[(2-bromoacetyl)amino]ethyl}-2-naphthyl)-1-cyclohexanecarboxamide,
* N-{8-[2-(heptanoylamino)ethyl]-2,6-dinaphthyl}-2-butenamide,
* N-{8-[2-(acetylamino)ethyl]-2-naphthyl}acetamide,
their enantiomers and diastereoisomers, and addition salts thereof with a pharmaceutically acceptable acid or base.

26. Compounds of formula (I) according to claim 1 that are :
* methyl N-{3-[2-(acetylamino)ethyl]benzo[*b*]furan-5-yl}carbamate,
* methyl 3-{2-[(cyclopropylcarbonyl)amino] ethyl}-1-benzofuran-5-yl-carbamate,
* tert-butyl 3-[2-(acetylamino)ethyl]-1-benzofuran-5-yl-carbamate,
* tert-butyl 3-[2-(acetylamino)ethyl]-1-benzofuran-5-yl-(methyl)carbamate,
* methyl 3-[2-(benzoylamino)ethyl]-1-benzofuran-5-yl-carbamate,
* methyl 3-[2-(isobutyrylamino)ethyl]-1-benzofuran-5-yl-carbamate,
* methyl 3-[2-(2-furoylamino)ethyl]-1-benzofuran-5-yl-carbamate,
* methyl 3- {2-[(cyclopentylcarbonyl)amino]ethyl}-1-benzofuran-5-yl-carbamate,
their enantiomers and diastereoisomers, and addition salts thereof with a pharmaceutically acceptable acid or base.

27. Compounds of formula (I) according to claim 1 that are :
* ethyl N-(8-{2-[(2-bromoacetyl)amino]ethyl}-2-naphthyl)carbamate,
* methyl N-{8-[2-(acetylamino)ethyl]-6-phenyl-2-naphthyl}carbamate,
* hexyl N-{8-[2-(acetylamino)ethyl]-5,6,7,8-tetrahydro-2-naphthyl}carbamate,
* methyl 8-[2-(acetylamino)ethyl]-2-naphthyl-carbamate,
their enantiomers and diastereoisomers, and addition salts thereof with a pharmaceutically acceptable acid or base.

28. Process for the preparation of compounds of formula (I), **characterised in that** there is used as starting material the compound of formula (X) : 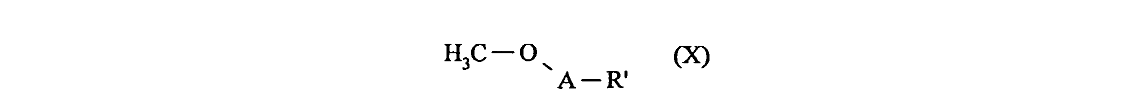 wherein A and R' are as defined in claim 1, which is subjected to demethylation using conventional agents such as HBr, AlCl₃, AlBr₃, BBr₃ or Lewis acid/nucleophile binary systems such as AlCl₃/PhCH₂SH, or BBr₃/Me₂S, for example, to obtain the compound of formula (XI) :
HO―A―R' (XI)
wherein A and R' are as defined hereinbefore,
which is converted, by means of the action of reagents such as POCl₃, PCl₅, Ph₃PBr₂, PhPCl₄, HBr or HI, into the corresponding halogenated compound of formula (XII) :
Hal - A - R' (XII)
wherein A and R' are as defined hereinbefore and Hal represents a halogen atom (which compounds of formula (XII) can be obtained by exchange reactions such as, for example, the treatment of a chlorinated compound with KF in dimethylformamide to yield the corresponding fluorinated compound or the treatment of a brominated compound with KI in the presence of copper salts to yield the corresponding iodinated compound),
which is treated :
with carbon monoxide and Bu₃SnH, the reaction being catalysed with palladium(0), to yield the corresponding aldehyde of formula (XIII) : 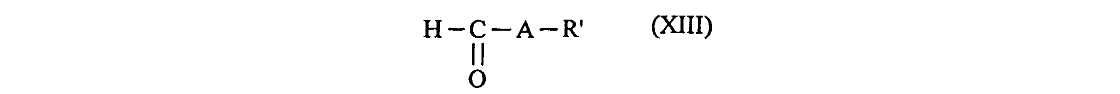 wherein A and R' are as defined hereinbefore,
which compound of formula (XIII) may alternatively be obtained by customary lithiation methods starting from the halogenated compound of formula (XII), or *via* the corresponding vinyl compound (obtained starting from the compound of formula (XII) by the action of vinyltributyltin and tetrakis palladium) subjected to ozonolysis, or furthermore by direct formylation of the nucleus A, for example according to a Vilsmeier reaction,
which compound of formula (XIII) is subjected to an oxidising agent to obtain the compound of formula (XIV):
HOOC ―A -R' (XVI)
wherein A and R' are as defined hereinbefore, which is:
converted, by the action of thionyl chloride and an azide, and then of an acid, into the compound of formula (XV) :
H₂N―A-R' (XV)
wherein A and R' are as defined hereinbefore, with which there is condensed:
- either an acyl chloride ClCORₐ or the corresponding anhydride (mixed or symmetrical), wherein Rₐ is as defined hereinbefore, to yield the compound of formula (I/c), a particular case of the compounds of formula (1) : 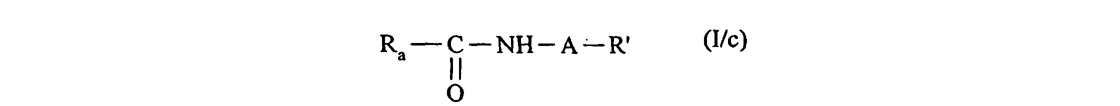 wherein Rₐ, A and R' are as defined hereinbefore,
which may be subjected to the action of a compound of formula (XVI) :
R¹ ₐ―J (XVI)
wherein R¹ₐ may take any of the values of Rₐ except for the hydrogen atom and J represents a leaving group such as a halogen atom or a tosyl group,
to obtain the compound of formula (I/d), a particular case of the compounds of formula (I) : 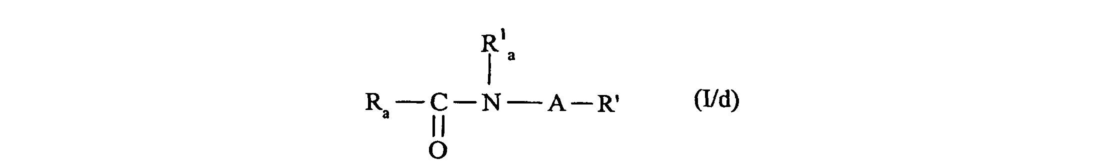 wherein Rₐ, R¹ₐ, A and R' are as defined hereinbefore,
which compounds of formulae (I/c) and (I/d) constitute the compound of formula (I/e), a particular case of the compounds of formula (I) : 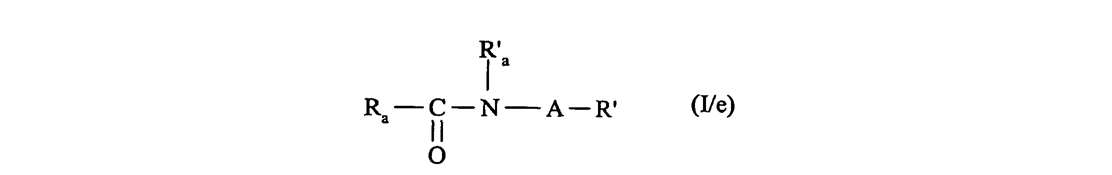 wherein Rₐ, R'ₐ, A and R' are as defined hereinbefore,
which compound of formula (I/e) may be subjected to a thionating agent, such as Lawesson's reagent, for example, to obtain the compound of formula (I/f), a particular case of the compounds of formula (I) : 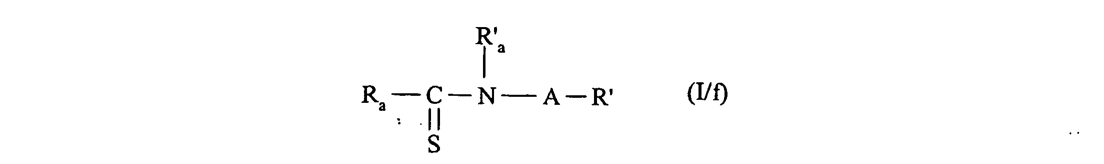 wherein Rₐ, R'ₐ, A and R' are as defined hereinbefore,
- or a compound of formula (XVII) :
Q=C=N-R'ₐ (XVII)
wherein Q and R'ₐ are as defined hereinbefore,
to yield the compound of formula (I/g), a particular case of the compounds of formula (I) : 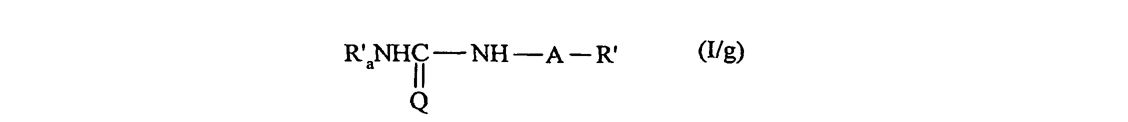 wherein R'ₐ, Q, A and R' are as defined hereinbefore,
which may be subjected to the action of a compound of formula (XVI) to obtain the compound of formula (I/h), a particular case of the compounds of formula (I) : 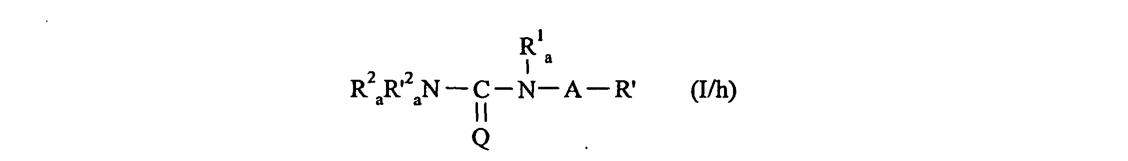 wherein Q, R¹ₐ, A and R' are as defined hereinbefore and R²ₐ and R'²ₐ, which may be the same or different, may take any of the values of Rₐ except for the hydrogen atom and cannot form a cyclic structure together with the nitrogen atom carrying them,
- or a compound of formula (XVIII) : 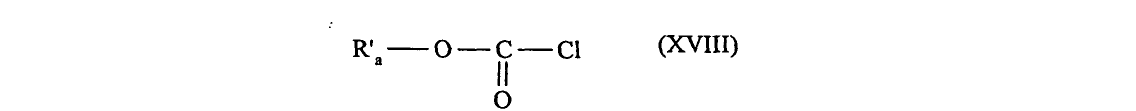 wherein R'ₐ is as defined hereinbefore, or its corresponding anhydride (R'ₐOCO)₂O,
to obtain the compound of formula (I/i), a particular case of the compounds of formula (I) : 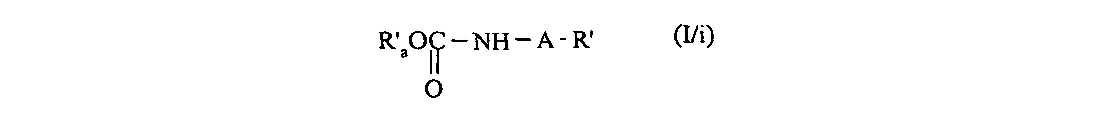 wherein R'ₐ, A et R' are as defined hereinbefore,
which may be subjected to the action of a compound of formula (XVI) and/or the action of a thionating agent to yield the compound of formula (I/j), a particular case of the compounds of formula (I) : 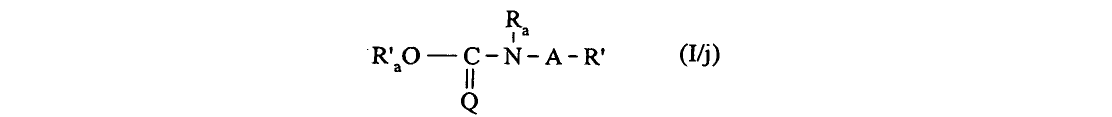 wherein Rₐ, R'ₐ, Q, A and R' are as defined hereinbefore,
- or a compound of formula (XIX) :
RₐSO₂Cl (XIX)
wherein Rₐ is as defined hereinbefore,
optionally followed by the action of a compound of formula (XVI) to yield the compound of formula (I/k), a particular case of the compounds of formula (I) : 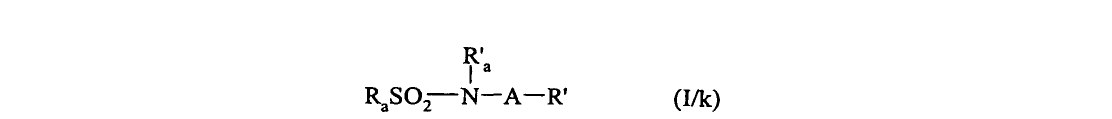 wherein Rₐ, R'ₐ, A and R' are as defined hereinbefore,
which compounds (I/c) to (I/k) can be purified in accordance with a conventional separation technique, are converted, if desired, into their addition salts with a pharmaceutically acceptable acid or base and, optionally, are separated into their isomers in accordance with a conventional separation technique.

29. Process for the preparation of compounds of formula (I) according to claim 1, **characterised in that** there is used as starting material the compound of formula (XXII) : 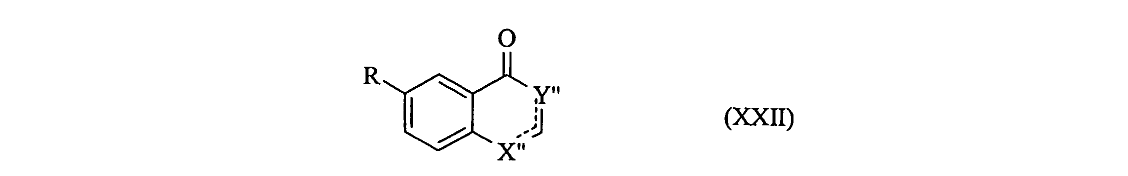 wherein R and the symbol .... are as defined in claim 1, and Y" represents a bond and **in that** case X" represents an oxygen or sulphur atom or a group NR₀ (wherein R₀ is as defined hereinbefore) or X" and Y", which are the same or different, represent a group C(H)_{q} (wherein q is 1 or 2),
which is subjected to a Wittig reaction and then to reduction to yield the compound of formula (XXIII) : 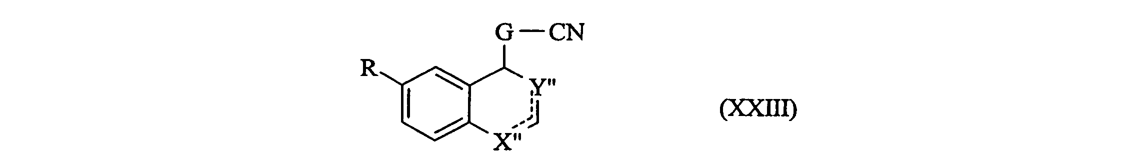 wherein R, X", Y", G and the symbol ..... are as defined hereinbefore,
which may be oxidised to yield the compound of formula (XXIV) : 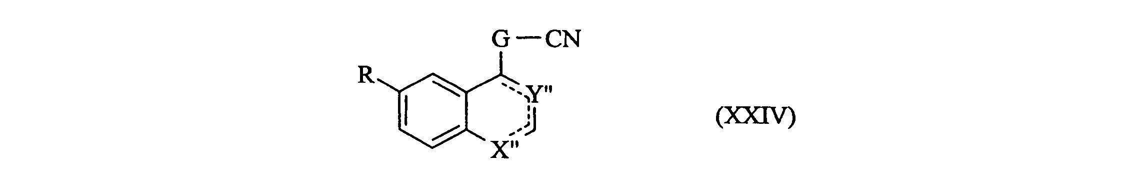 wherein R, X", Y", G and the symbol ..... are as defined hereinbefore, which is :
* either hydrolysed in an acid or basic medium and then subjected, after activation to the acid chloride form or in the presence of a coupling agent, to the action of an amine HNR'ₐR"ₐ wherein R'ₐ and R"ₐ are as defined hereinbefore to yield the compound of formula (I/p), a particular case of the compounds of formula (I) : 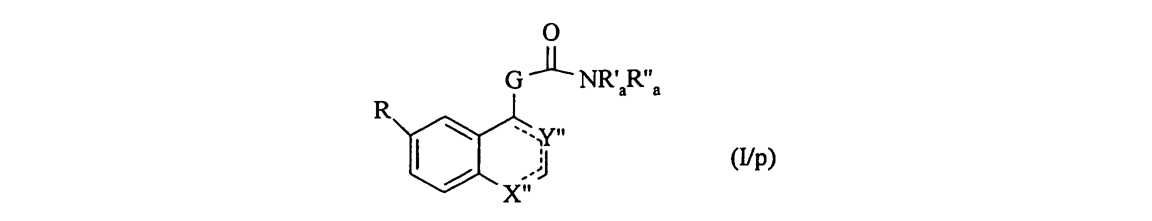 wherein R, X", Y", G, R'ₐ, R"ₐ and the symbol ..... are as defined hereinbefore,
which may be subjected to a thionating agent such as Lawesson's reagent to yield the compound of formula (I/q), a particular case of the compounds of formula (I) : 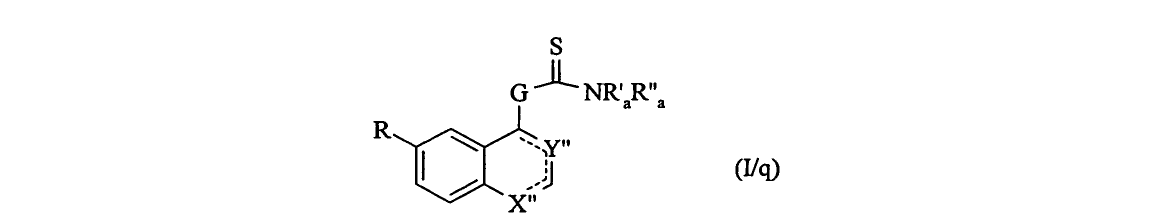 wherein R, X", Y", G, R'ₐ, R"ₐ and the symbol ..... are as defined hereinbefore,
* or hydrolysed in an acid or basic medium and then converted into the corresponding azide to yield, after having been subjected to a Curtius rearrangement and hydrolysis, the compound of formula (XXV) : 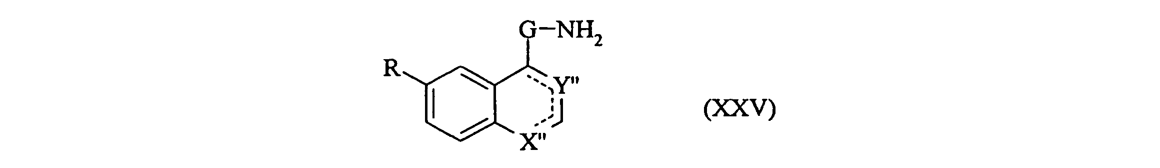
wherein R, X", Y" and G are as defined hereinbefore,
which is reacted with :
- an acyl chloride CICOR'ₐ or the corresponding anhydride (mixed or symmetrical) wherein R'ₐ is as defined hereinbefore, optionally followed by the action of a compound of formula (XVI) and/or the action of a thionating agent to yield the compound of formula (I/r), a particular case of the compounds of formula (1) : 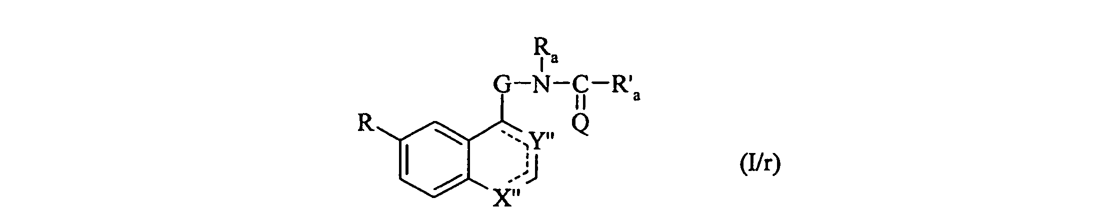 wherein R, X", Y", G, Rₐ, R'ₐ, Q and the symbol .... are as defined hereinbefore,
- or with a compound of formula (XVII), optionally followed by the action of a compound of formula (XVI) to yield the compound of formula (I/s), a particular case of the compounds of formula (I) : 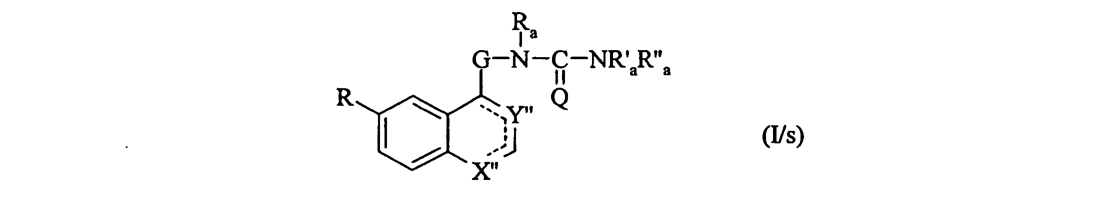
wherein R, X", Y", G, Rₐ, R'ₐ, R"ₐ, Q and the symbol ... are as defined hereinbefore,
which compounds (I/p) to (I/s) can be purified in accordance with a conventional separation technique, are converted, if desired, into their addition salts with a pharmaceutically acceptable acid or base and, optionally, are separated into their isomers in accordance with a conventional separation technique.

30. Pharmaceutical compositions comprising compounds of formula (I) according to any one of claims 1 to 27 or an addition salt thereof with a pharmaceutically acceptable acid or base, in combination with one or more pharmaceutically acceptable excipients.

31. Pharmaceutical compositions according to claim 30 for use in the manufacture of medicaments for the treatment of disorders associated with the melatoninergic system.
